# EUROPEAN PATENT APPLICATION

(11) **EP 2 740 491 A1**
(43) Date of publication of application: **11.06.2014**
(21) Application number: 12306524.5
(22) Date of filing: 05.12.2012
(51) Int. Cl.: A61K 41/00, A61K 47/48, A61K 49/00, A61K 49/10

(54) **Conjugates of the B-subunit of shiga toxin for use as contrasting agents for imaging and therapy**

(71) Applicant: Institut Curie, 75005 Paris (FR); Centre National de la Recherche Scientifique, 75794 Paris Cedex 16 (FR)
(72) Inventor: The designation of the inventor has not yet been filed
(74) Representative: Paris, Fabienne

(57) **Abstract**

Multivalent conjugates comprising the following formula: (STxB-linker A-S)ₓ-GNS-(S-linker B-T)_{y} wherein STxB is the B-subunit of Shiga toxin; linker A is a noncleavable linker; linker B is a cleavable linker used to release at least one T or a noncleavable linker; GNS is a gold nanostructure; S is a sulfhydryl group and x and y can vary from 1 to 8,000; and T is a molecule selected from the group of: contrast agents, cytotoxic agents, prodrugs and antigens and x and y can vary from 1 to 10,000. These multivalent conjugates can be used in therapy to treat cancer and in medical imaging. Methods to distinguish normal cells from abnormal cells and methods for treating cancer by photothermal therapy or photothermal therapy and cytotoxic therapy are also part of the present invention.

SEQUENCE LISTING

<110>  INSTITUT CURIE
       CENTRE NATIONAL DE LA RECHERCHE SCIENTIFIQUE

<120>  CONJUGATES OF THE B SUBUNIT OF SHIGA TOXIN FOR USE AS CONTRASTING
        AGENTS FOR IMAGING AND THERAPY

<130>  B10066-JAZ/SDU

<140>  EP XXXXXXX.X
<141>  2012-12-05

<160>  1

<170>  PatentIn version 3.5

<210>  1
<211>  90
<212>  PRT
<213>  Artificial Sequence

<220>
<223>  B subunit of Shiga toxin

<400>  1

Met Lys Lys Thr Leu Leu Ile Ala Ala Ser Leu Ser Phe Phe Ser Ala
1               5                   10                  15

Ser Ala Leu Ala Thr Pro Asp Cys Val Thr Gly Lys Val Glu Tyr Thr
            20                  25                  30

Lys Tyr Asn Asp Asp Asp Thr Phe Thr Val Lys Val Gly Asp Lys Glu
        35                  40                  45

Leu Phe Thr Asn Arg Trp Asn Leu Gln Ser Leu Leu Leu Ser Ala Gln
    50                  55                  60

Ile Thr Gly Met Thr Val Thr Ile Lys Thr Asn Ala Cys His Asn Gly
65                  70                  75                  80

Gly Gly Phe Ser Glu Val Ile Phe Arg Cys
                85                  90

## Description

### TECHNICAL FIELD

The present invention relates to multivalent conjugates comprising the following formula: (STxB-linker A-S)ₓ-GNS-(S-linker B-T)_{y} wherein STxB is the B- subunit of Shiga toxin; linker A is a noncleavable linker; linker B is a cleavable linker used to release at least one T or a noncleavable linker; GNS is a gold nanostructure; S is a sulfhydryl group and x and y can vary from 1 to 10,000 and T is a molecule selected from the group of: contrast agents, cytotoxic agents, prodrugs and antigens. These multivalent conjugates can be used in therapy to treat cancer and in medical imaging. Methods to distinguish normal cells from abnormal cells and methods for treating cancer by photothermal therapy or photothermal therapy and cytotoxic therapy are also part of the present invention.

### Background of the Invention

Magnetic Resonance Imaging (MRI) works by surrounding a patient with a powerful magnetic field, which aligns the nuclei of hydrogen atoms in the body. Radio wave pulses systematically probe the tissues of interest knocking those atoms out of alignment, causing the atoms to spin. The radio frequency pulse is then stopped causing the protons in hydrogen atoms to relax back into their previous state via two relaxation processes, namely T1 and T2. The hydrogen protons emit a radio frequency signal that can be detected and translated into an image.

The signal intensity is proportional to the amount of hydrogen protons and the more protons, the brighter the image. The MRI can depict longitudinal relaxation movement, which is T1 that has a spin-lattice relaxation time or T2, which is a transverse relaxation movement that has a spin-spin relaxation time. Variations in strength of the magnetic field, orientation and timing of the radiofrequency pulses can also be adjusted depending on the nature of the scan.

Near-infrared fluorescence is also a technique that can be used in medical imaging. This technique is based on the property of certain molecules to absorb light at one wavelength and to emit light at another wavelength. Tomographic near-infrared fluorescence imaging is a three dimensional imaging technique that can create images similar to MRI.

Contrast agents are chemical substances that can be introduced into the different regions of the body for three dimensional imaging or therapeutic applications to increase the differences between different tissues or between normal tissues and abnormal tissues by altering the relaxation times. MRI contrast agents are classified by the different changes in the relaxation times after injection. There are positive contrast agents, which cause a reduction in T1 relaxation time and thus increase signal intensity on T1 weighted images and appear bright with an improved contrast on MRI. In contrast, there are negative contrast agents, which result in shorter T1 and T2 relaxation times and appear predominantly dark on MRI.

Positive contrast agents are typically small molecular weight compounds containing as their active element Gadolinium, Manganese or Iron, which have unpaired electron spins in their outer shells and long relaxivities. Negative contrast agents are generally small particle aggregates of superparamagnetic iron oxide and produce mainly spin spin relaxation effects. Superparamagnetic and ultrasmall superparamagnetic iron oxides generally consist of a crystalline iron oxide core containing thousands of iron atoms and a shell of polymer, dextran or polyethyleneglycol and produce high T2 relaxivities. Ultrasmall superparamagnetic iron oxides that are smaller than 300 nm cause a substantial T1 relaxation and T2 weighted effects are predominant.

Near-infrared fluorescence contrast agents must have excitation and emission maxima in the near-infrared range between 700 to 900 nm; high quantum yield; chemical and optical stability; and suitable pharmaceutical properties such as aqueous solubility, low non-specific binding, rapid clearance of the free dye and low toxicity.

The contrast agents used for MRI and near-infrared fluorescence are usually administered intravenously and should have a high aqueous solubility, should be biocompatible and should be eliminated from the body after analysis. The complexes of Gadolinium ion (Gd³⁺) are contrast agents that are well known in the art for augmenting the intensity of the signal for use in MRI and near-infrared fluorescence. However, a high concentration must be used since Gd³⁺ is non-specific, is a weak contrasting agent and their elimination from the kidneys is quite rapid. Therefore, other solutions to improve MRI and near-infrared fluorescence imaging were attempted in this art.

For instance, with the onset of nanotechnology, nanoparticles and nanorods were developed. Liu et al discloses the use of biocompatible gold nanorods that can be fabricated as multimodal probes for cancer labeling *in vivo* (Liu et al Plasmonics (2011) 6:105-112). These gold nanorods were fabricated with PEG/PEG-Tf/PEG-NH₂-GD-coated nanorods. The efficacy of targeted delivery of these coated gold nanorods and multimodal imaging had yet to be tested in mice models.

Huang et al disclose cancer imaging and photothermal therapy in the near-infrared region using gold nanorods that were conjugated to anti-epidermal growth factor receptor (EGFR) monoclonal antibodies (Huang et al J. Am Chem Soc. (2006) 128:1999-2004.

U.S. Patent 7,829,065 discloses anti-CD74 immunoconjugates that are used for therapeutic and/or diagnostic agents. These immunoconjugates are PEG-lipid conjugates incorporated into liposomes.

However, there are drawbacks using monoclonal antibodies in immunoconjugates since in many instances they do not bind to their target cell since tumor blood flow is not always optimal and high interstitial pressure within the tumor can prevent the monoclonal antibodies from binding. Furthermore, monoclonal antibodies translate into lysosomes once they enter the cell which can quickly degrade the contrast agent.

The B-subunit of Shiga toxin (STxB) is nontoxic and exhibits high specificity for cancer cells expressing the cell surface glycosphingolipid receptor, globotriaosyl ceramide, abbreviated as Gb3. Once targeted to Gb3 cells, it undergoes cellular internalization and is transported in a retrograde fashion from the plasma membrane to the endoplasmic reticulum via early endosomes and the *trans*-Golgi network. This specific targeting avoids STxB recycling and degradation in the lysosomes and could be detected in the tumor cells even five days after uptake. (Falguières et al Molecular Cancer Therapeutics 2008;7:2498-2508.

Gb3 expression has been shown in Burkitt's and centrofollicular lymphomas (Oosterwijk et al Int. J. Cancer 1991;48:848-54), in solid breast and solid ovarian tumors (LaCasse et al,Blood 1999; 94:2901-2010; Arab et al Oncl Res 1997;9:553-63) and in testicular seminomas (Ohyama et al Int, J. Cancer 1990;45:1040-4. Tumor cells of epithelial origin, as well as tumor-associated blood vessels and stroma are the origin of increased Gb3 expression in colorectal carcinoma (Falguières et al Mol. Cancer Ther 2009;7:2498-508). In Maak et al Mol Cancer Ther 2011;10:10:1918-28, tumor-specific targeting of pancreatic cancer having Gb3 expression was demonstrated.

The B-subunit of Shiga toxin, a toxin vector, is known to be a universal carrier that can be used to target directly or indirectly the Gb3 receptor. U.S. Patent No. 6,613,882 describes a chimeric polypeptide of the formula B-X, where B is the B- subunit of Shiga toxin or functional equivalent thereof and X is a polypeptide of therapeutic significance. U.S. Patent 7,632,514 B2 describes a carrier having the formula STxB-Z (n)-Cys-X where STxB is the B-subunit of Shiga toxin Z is an amino acid linker without sulfhydryl groups and n is 0, 1, 2 or a polypeptide and Cys is Cysteine.

Although the B-subunit of Shiga toxin was known to act as a carrier to deliver pharmaceutically active principles to cancer cells, it was unpredictable whether this carrier could indeed be conjugated with a contrast agent to be used in MRI or near-infrared fluorescence (NIF) imaging and photothermal therapy to augment the contrasting of the images obtained by MRI and NIF and diminish the quantity of contrast agent that needs to be injected.

Indeed, there still remains in this art a need for more efficient contrast agents that can augment the intensity signal, have a high aqueous solubility, are biocompatible, have less side effects and can be completely eliminated from the body after analysis with no adverse side effects. There is also a need in the art for methods not only to image, but also to treat cancer cells.

### Summary of the Invention

The present invention provides a multivalent conjugate comprising the following formula:

(STxB-linker A-S)ₓ-GNS-(S-linker B-T)_{y}

wherein STxB is the B-subunit of Shiga toxin; linker A is a noncleavable linker; linker B is a cleavable linker used to release at least one T or a noncleavable linker; GNS is a gold nanostructure; S is a sulfhydryl group and x and y can vary from 1 to 10,000; and T is a molecule selected from the group of: contrast agents, cytotoxic agents, prodrugs and antigens.

In another aspect of the present invention the noncleavable linker A is selected from the group comprising polyethylene glycols, polyacylamides, polysaccharides, acrylates, methacrylates, polyvinylalcohols, polyvinylpyrrolidones, polystyrenes, polysulfobetaines and mixtures thereof, in the multivalent conjugate.

The cleavable linker B is selected from the group comprising 3-maleimidobenzoic acid N-hydroxyysuccinimide ester (MBS), N-succinimidyl 3-[2-pyridyldithio]-propionate (SPDP), [S-(N-succinimidyl)thioacetate] (SATA), [(N-succinimidiyloxy carbonyl)1-methyl-1-(2-pyridyldithio) toluene] (SMPT), 2-iminothiolane (2-IT), (Sulfosuccinimidyl N-[3-(Acetylthio)-3-methylbutyryl)-beta-alanine]) (sulfoNHS-ATMBA), thioimidates such as AMPT and M-CDPT and 2-[N-chlorocarbonyl)-N-methylamino]1-ethyl 2-pyridyldisulfide and a maleimide linker..

In another aspect the present invention comprises a contrast agent selected from the group comprising indocyanine green (IcG), 1,1'-bis-(4-sulfobutyl) indotricarbocyanine-5,5'-dicarboxylic acid diglucamide monosodium salt (SIDAG),disulfated indocyanine, tetra-sulfated indocyanine, cyanine dyes, Cy7, Cy5.5, XenoLight CF680, Xenolight CF 750, Xenolight CF 770, AlexaFluor^{®} 647, AlexaFluor^{®} 680, AlexaFluor^{®} 700, AlexaFluor^{®} 750, DyLight 650, DyLight 680, DyLight 750, DyLight 755, heptamethine cyanine, positron emitting radioisotopes, gamma-emitting radioisotopes and mixtures thereof.

The cytotoxic agent in the present invention can be selected from the group comprising halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, auristatin, auristatin E and monomethyl auristatin E, methotrexate, platinum drugs and derivatives thereof, pharmaceutically acceptable salts thereof and mixtures thereof.

In yet another aspect the prodrugs of the present invention can be selected from the group comprising INNO-206 (a 6-maleimidocaproyl hydrazone derivative of doxorubicin), cyclophosphamide, capecitabine, dacarbazine, prodrugs of 5-fluorouracil, etoposide phosphate prodrugs, gemcitabine phosphoramidate prodrug, irinotecan and mixtures thereof.

Antigens, of the present invention, can be selected from the group comprising WT1,MUC1, LMP2, HPV E6, E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinase3, bcr-abl, Tyrosinase, Survivin, PSA, hTERT, Sarcoma translocation breakpoints, EphA2, PAP, ML-IAP, AFP, EpCAM, ERG, NA17, PAX3, ALK, androgen receptor, Cyclin B1, Polysialic acid, MYCN, RhoC, TRP-2, GD3, Fucosyl GM1, Mesothelin, PSCA, MAGE A1, sLe, CYP1b1, PLAC1, GM3m, BORIS, Tn, MAGE-B2, DAM-10, SAGE, RAGE and mixtures thereof.

In another embodiment in the multivalent conjugate the gold nanostructure (GNS) are functionalized with sulfhydryl groups, methyl groups, carboxylic acid groups or amine groups and /or have present at their surface between 1 to 100,000 amine groups.

The functional equivalent of the Shiga B toxin can be selected from the group of Shiga-like toxin 1, Shiga-like toxin 2, Shiga-like toxin 2c, Shiga-like toxin 2d1, Shiga-like toxin 2d2, Shiga-like toxin 2e, Shiga-like toxin 2f and Shiga-like toxin 2y,verotoxin-1, verotoxin-2, verotoxin 2c or verotoxin 2v..

In another embodiment the multivalent conjugate, as described herein, is used for distinguishing normal cells from abnormal cells. The normal cells and abnormal cells can be distinguished by magnetic resonance imaging or near-infrared fluorescence imaging.

The multivalent conjugate, as described herein, for use as a medicament preferably for use in treating cancer cells.

The multivalent conjugate, as described herein, can be used for the preparation of a medicament to treat cancer cells is yet another aspect of the present invention.

The cancer cells can be treated or distinguished using one of the following:
(1) photothermal therapy,
(2) imaging using near-infrared fluorescence or MRI or Positron emission tomography (PET) or Single photo emission computed tomography (SPECT),
(3) photothermal therapy and administering cytotoxic agents or vice versa,
(4) imaging using near-infrared fluorescence or MRI and administering cytotoxic agents or vice versa,
(5) photothermal therapy and administering cancer antigens or vice versa, or
(6) imaging using near-infrared fluorescence or MRI and administering cancer antigens or vice versa.

The photothermal therapy, as described herein, involves first imaging said cancer cells using a magnetic resonance device or a near-infrared fluorescence device and secondly heating said cancer cells using near infrared irradiation.

In yet another aspect the present invention provides a multivalent conjugate, as described herein, for use in diagnosis for use in imaging cancer cells.

The abnormal cells or cancer cells can be selected from the group comprising acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ,* embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ,* lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vaginal cancer and vulvar cancer.

In yet another embodiment a pharmaceutical composition is provided comprising a first multivalent conjugate, as described herein, wherein T is a cytotoxic agent or a contrast agent and a second multivalent conjugate, as described herein, wherein T is a cancer antigen and a pharmaceutically acceptable vehicle.

Other aspects and embodiments are set forth below, or will readily arise from the following description of the preferred embodiments.

### Brief Description of the Figures

Fig. 1 are photographs showing the internalization of STxB-(Gd-DOTA)₅ in HeLa cells.

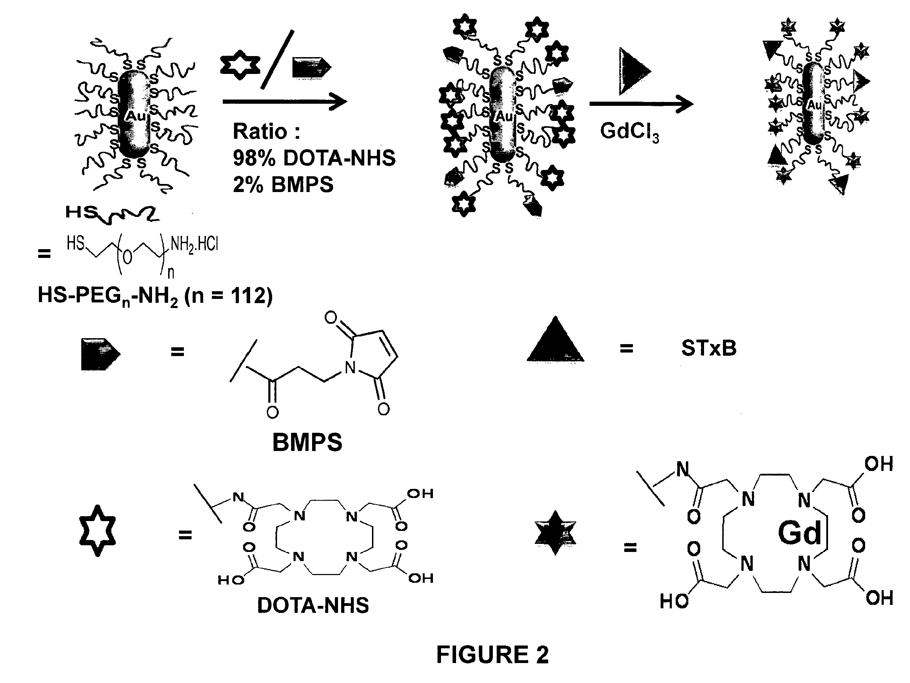

Fig. 2 is a schematic representation of how the STxB and DOTA-NHS are coupled to the gold nanorods.

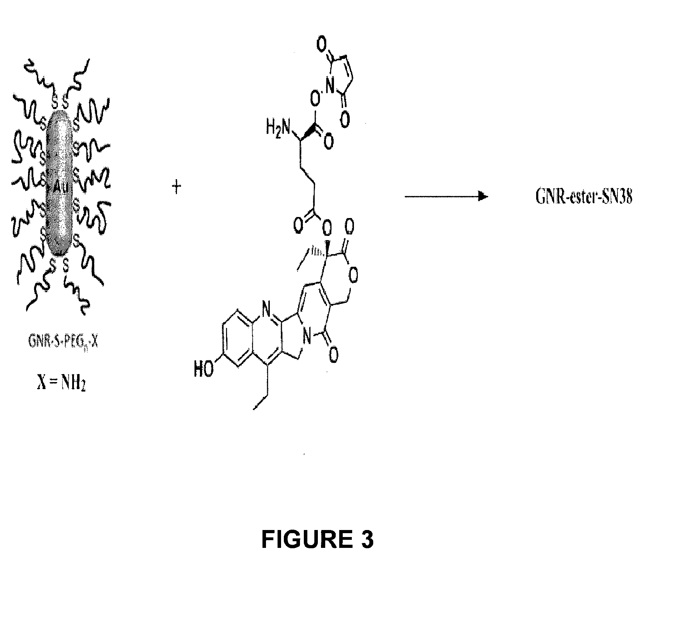

Fig. 3 is a schematic of a prodrug-GNS targeted with the Shiga B-subunit.
Fig 4 is a picture of mice that have been injected with GNR-CF680-STxB conjugates, 1 hour (top set of mice) and 24 hours (middle set of mice) and control after 6 hours (bottom set of mice) after injection.

### Description of the Preferred Embodiments

By "retrograde transport" is meant that the B-subunit enters the cell after binding to the Gb3 receptor through a clathrin-independent endocytosis, reaches the interface between the early endosome and the *trans-*Golgi network and is transported to the Golgi via the retrograde pathway and is transported from the Golgi to the endoplasmic reticulum. The retrograde pathway is distinct from the well known pathway used by the mannose-6-phosphate receptor.

The abbreviation "Gb3" receptor means the globotriasylceramide receptor.

The term "warm blooded animals" as used herein includes birds and mammals.

Examples of birds that can be treated with the compositions and methods of the invention include blue birds, cardinals, doves, eagles, geese, turkeys, chickens, hens, ducks, quails, herons, sparrows, woodpeckers, owls, parrots and the like.

The term "mammal" encompasses any of various warm-blooded vertebrate animals of the class Mammalia, including humans, characterized by a covering of hair on the skin and, in the female, milk-producing mammary glands for nourishing the young. The present invention is not limited to treating humans, but also encompasses veterinary applications, especially since it is well known that animals also can have different medical pathologies.

Examples of mammals that can be treated with the compositions and methods of the present invention include humans, domestic animals such as dogs and cats, horses, mice, goats, deer, cows, rabbits, zoo animals, bears, monkeys, apes, elks, bison, although this invention may be applied to other mammalian species as well.

By "functional equivalent thereof" with respect to the B-subunit of Shiga toxin embodiment is meant that the toxin acts immunogenically like the B-subunit of Shiga toxin and can bind to Gb3 cells and/or they can internalize the at least one antigen such that the at least one antigen can be presented to the MHC class I pathway or the MHC class I and class II pathways on the same antigen presenting cells. Furthermore, the functional equivalents can target dendritic cells with respect to antigen administration.

"Treating," as used herein means giving medical care or attention to a warm blooded animal and/or the combating of a disease, especially cancer, via the use of the multivalent conjugates as described herein, as well as the methods of treatment, as described herein.

By "consisting essentially of" means that the major elements are present in the multivalent conjugate, but also minor ingredients that do not materially affect the conjugate or multivalent conjugate also be present.

The terms, comprising, consisting of and consisting essentially of can be interchanged throughout the specification.

By the term "conjugate" is meant at least two entities that are joined together, linked together or coupled together.

"Multivalent conjugate" means a conjugate that has a valence of three or higher.

By "contrast agent" is meant a substance used to enhance the contrast of structures or fluids within the body in medical imaging.

"Nanostructures" as used herein include gold nanoparticles, gold nanoshells, gold nanocages, gold nanorods and the like.

"Nanorods" as used herein means nanostructures shaped like sticks with a diameter in the nanoscale but having a much longer length. The longitudinal axis is also in nanoscale. Nanorods are of one morphology and each of their dimensions range from 1 nm to 100 nm or from 20 nm to 400 nm. Nanorods are produced by direct chemical synthesis.

By "functionalized nanostructures" or "functionalized nanorods" is meant that the surface of the nanostructures or nanorods is altered such that hydrophilic moieties that prevent aggregation and maintain a superior stability in the warm blooded animal body is achieved. In one aspect, the surface of the nanostructures or nanorods are functionalized with sulfhydryl groups, methyl groups, carboxylic acid groups or amine groups.

The term "photothermal therapy" involves localizing the cancer cells in the warm blooded animal and applying heat to the cancer cells using near infrared irradiation.

The term "vice versa" as used herein, means reversing the order of items just mentioned.

More specifically the present invention encompasses a multivalent conjugate comprising the following formula:

(STxB-linker A-S)ₓ-GNS-(S-linker B-T)_{y}

wherein STxB is the B-subunit of Shiga toxin; linker A is a noncleavable linker; linker B is a cleavable linker used to release at least one T or noncleavable linker; GNS is a gold nanostructure; S is a sulfhydryl group and x and y can vary from 1 to 10,000; and T is a molecule selected from the group of: contrast agents, cytotoxic agents, prodrugs and antigens. The antigens can be cancer antigens or non-cancer antigens.

The B-subunit of Shiga toxin acts as a carrier for the multivalent conjugate, as described herein, and targets cancer cells. By targeting cancer cells and more specifically the Gb3 receptor found on cancer cells and in the vasculature of cancer cells, the multivalent conjugates, as described herein, have sufficient cellular uptake, low cytotoxicity and especially low cytotoxicity for the healthy non-cancerous cells and few systemic and off-target effects. Furthermore, Gb3 is highly expressed in tumors with more than 10⁷ binding sites per cell.

The sequence of the B-subunit of Shiga toxin has been described in Strockbine et al., J. Bacteriol, 170, 1116-22 (1988). The functional equivalent is one in which the toxin can bind to the Gb3 receptor and/or causes the internalization of an antigen and its presentation to the MHC class I pathway or both MHC class I and class II pathways. Thus, the B-subunits of Shiga-like toxins from *E. coli* such as Shiga-like toxin 1, Shiga-like toxin 2 and the Shiga-like toxin 2 variants such as Shiga-like toxin 2c, Shiga-like toxin 2d1, Shiga-like toxin 2d2, Shiga-like toxin 2e, Shiga-like toxin 2f and Shiga-like toxin 2y can all be considered functional equivalents. In an embodiment the B-subunits of verotoxin-1 or verotoxin-2 or verotoxin 2c or verotoxin 2v from *E. coli* can be used instead of the B-subunit of Shiga toxin in the formulation of the multivalent conjugate or pharmaceutical composition of the present invention. It should be noted that verotoxin-1 is an alternative name for Shiga-like toxin 1 and that verotoxin-2 is an alternative name for Shiga-like toxin 2.

The B-subunit of Shiga toxin or the functional equivalent thereof is devoid of toxin activity and hence is not toxic to mammals. Lacking toxicity does not affect the binding of the B-subunit of Shiga toxin or functional equivalents thereof to Gb3 receptors or the entry into the MHC class I or MHC class I or II pathways in the case of administering antigens.

The B-subunit of Shiga toxin or functional equivalent thereof, once binding to the Gb3 receptor and entering the cell follows retrograde transport within the cell. This retrograde transport avoids lysosomal destruction of the multivalent conjugate.

The binding to the Gb3 receptor may be evaluated by methods known in the art such as those described by Tarrago-Trani in Protein Extraction and Purification, 39:pp 170-176 (2004) or Nishikawa et al in Chem Pharm Bull April 54(4): pp.522-7 (2006), which are incorporated herein by reference.

In one embodiment the B-subunit of Shiga toxin has the sequence:
COOH-MKKTLLIAASLSFFSASALATPDCVTGKVEYTKYNDDDTFTVKVGDK ELFTNRWNLQSLLLSAQITGMTVTIKTNACHNGGGFSEVIFRC-NH2 (SEQ ID NO: 1).

In the multivalent conjugate, as described herein, or the pharmaceutical composition, as described herein, the functional equivalent of the Shiga B toxin is selected from the group of Shiga-like toxin 1, Shiga-like toxin 2 , Shiga-like toxin 2c, Shiga-like toxin 2d1, Shiga-like toxin 2d2, Shiga-like toxin 2e, Shiga-like toxin 2f and Shiga-like toxin 2y,verotoxin-1, verotoxin-2, verotoxin 2c or verotoxin 2v.

A is a noncleavable linker, which is attached to the gold nanostructure through a sulfhydryl group, a methyl group, a carboxylic acid group or amine group. Linker A is a polymer, to which the B-subunit of Shiga toxin is attached. Any polymer can be used that is compatible with the multivalent conjugate and its delivery of contrast agents, cytotoxic agents, prodrugs and antigens into cancer cells. Generally linker A is selected from the group comprising polyethylene glycols, polyacylamides, polysaccharides, acrylates, methacrylates, polyvinylalcohols, polyvinylpyrrolidones, polystyrenes, polysulfobetaines and mixtures thereof.

The multivalent conjugate also comprises a cleavable or noncleavable linker B, which is coupled to the gold nanostructure through a sulfhydyl group, at one end and at the other end is coupled to contrast agents, cytotoxic agents, prodrugs and antigens. Any linker can be used such as thiol linkers. Examples of other linkers include 3-maleimidobenzoic acid N-hydroxyysuccinimide ester (MBS), N-succinimidyl 3-[2-pyridyldithio]-propionate (SPDP), [S-(N-succinimidyl)thioacetate] (SATA), [(N-succinimidiyloxy carbonyl)1-methyl-1-(2-pyridyldithio) toluene] (SMPT), 2-iminothiolane (2-IT), (Sulfosuccinimidyl N-[3-(Acetylthio)-3-methylbutyryl)-beta-alanine]) (suIfoNHS-ATMBA), thioimidates such as AMPT and M-CDPT and 2-[N-chlorocarbonyl)-N-methylamino]1-ethyl 2-pyridyldisulfide, peptidic linkers, linkers having esters, linkers having carbamates and a maleimide linker. In one aspect the linker is a maleimide linker.

The noncleavable linker in linker B can be a polymer such as polyethylene glycols, polyacylamides, polysaccharides, acrylates, methacrylates, polyvinylalcohols, polyvinylpyrrolidones, polystyrenes, polysulfobetaines and mixtures thereof, a maleimidocaproyl linker, carbodiimide linker, a MalC linker, a thioether linker and the like.

In another aspect, the gold nanostructure (GNS) can be amine functionalized and coupled with the linkers sulfosuccinimidyl-4-[N-malermidomethyl] cyclohexan-1-carboxylate (SMCC), N-hydroxy succinimide-polyethylene glycol-maleimide (NHS-PEG-MAL), N-succinimidyl 3-[2-pyridyldithio (SPDP) or N-succinimidyl iodoacetate (SIA).

In yet another aspect, the gold nanostructure (GNS) can also be carboxyl-functionalized using an N-hydroxy succinimide (NHS) ester intermediate.

In another aspect linker B can be coupled to the gold nanostructures, preferably nanorods, through a maleimide, SPDP or bromacetic acid functional group.

The linker B can be cleaved via glutathione with those linkers having a disulfide bonds or via esterases or proteases for those linkers having esters, carbamates and peptidic bonds. This cleavage takes place once the multivalent conjugate is delivered into the cells.

The multivalent conjugate, as described herein, can have a contrast agent coupled thereto. This contrast agent can be any near-infrared contrast agent that can be used in near-infrared fluorescence and magnetic resonance imaging. Examples of the contrast agents that can be used in the multivalent conjugate, as described herein, include indocyanine green (IcG), 1,1'-bis-(4-sulfobutyl) indotricarbocyanine-5,5'-dicarboxylic acid diglucamide monosodium salt (SIDAG), disulfated indocyanine, tetra-sulfated indocyanine, any cyanine dyes such as Cy7, Cy5.5, XenoLight CF680, Xenolight CF 750, Xenolight CF 770, AlexaFluor^{®} 647, AlexaFluor^{®} 680, AlexaFluor^{®} 700, AlexaFluor^{®} 750, DyLight 650, DyLight 680, DyLight 750, DyLight 755, heptamethine cyanine and mixtures thereof.

Positron emitting radioisotopes and gamma-emitting radioisotopes can also be used as a contrast agent. Examples of positron emitting radioisotopes are ¹⁵O, ¹³N, ¹¹C, ¹⁸F and ¹²⁴I. Examples of gamma-emitting radioisotopes include ^{99m}Tc, ¹¹¹I, ¹²³I, ¹³¹I and ¹²⁵I.

In another aspect a multivalent conjugate comprising functionalized gold nanostructures as a support coupled to at least one B-subunit of Shiga toxin or a functional equivalent thereof and coupled to at least one contrast agent comprising a gadolinium ion (Gd³⁺) is provided. The B-subunit of Shiga toxin or the functional equivalents thereof are described herein.

In another embodiment the multivalent conjugate has a contrast agent selected from the group comprising 1,4,7,10-tetraazacyclododecane-1,4,7,10-tetraacteic acid-gadolinium (DOTA-Gd³⁺),diethylenetriaminepentaacetic acid- gadolinium (DTPA-Gd³⁺), triethylenetetramine-gadolinium (TETA-Gd³⁺) and 1,4,7-triazacyclononane-1,4,7-trisacetic acid-gadolinium (NOTA-Gd³⁺). In an embodiment the contrast agent is 1,4,7,10-tetraazacyclododecane-1,4,7,10-tetraacteic acid-gadolinium (DOTA-Gd³⁺).

Cytotoxic agents that can be used in the multivalent conjugate, as described herein, can be antimitotic agents selected from the group comprising at least one antimitotic agent that has a reactive hydroxyl group selected from the group comprising halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, auristatin, auristatin E and monomethyl auristatin E, methotrexate, pharmaceutically acceptable salts thereof and mixtures thereof.

Also platinum drugs and derivatives thereof is yet another category of cytotoxic agents that can be used in the multivalent conjugate, as described herein. In this regard, the platinum drugs include cisplatinum, carboplatin, oxiplatin, platinum (IV), mixtures thereof, derivatives thereof, pharmaceutically acceptable salts thereof and the like.

The pharmaceutically acceptable salts of the antimitotic agents that are used in the conjugates, described herein, and the pharmaceutical compositions, described herein, include those that are organic or inorganic salts of the antimitotic agents. These are well known and described in the Physician's Desk Reference, The Merck Index and Goodman and Gilman's The Pharmacological Basis of Therapeutics. The pharmaceutically acceptable salts are, for example, sodium, potassium, ammonium, calcium and magnesium and salts formed with inorganic acids such as hydrochloric acid, hydrobromic acid, phosphoric acid, sulfuric acid and the like or salts formed with organic acids such as oxalic acid, fumaric acid, tartaric acid, malonic acid, acetic acid, citric acid, benzoic acid and the like.

In another aspect, T in the multivalent conjugate can be a prodrug. Any prodrug can be used in the present invention which can be converted in the body of a warm blooded animal to a cancer drug. Examples of the prodrugs that can be used in the present invention include INNO-206 (a 6-maleimidocaproyl hydrazone derivative of doxorubicin), cyclophosphamide, capecitabine, dacarbazine, prodrugs of 5-fluorouracil, etoposide phosphate prodrugs, gemcitabine phosphoramidate prodrug, irinotecan and mixtures thereof.

The prodrug can be coupled to the GNS by two methods. The SH-linker-B-prodrug can be coupled by direct reaction of the SH-linker-B-prodrug with gold through the fixation of a terminal cysteine or methionine to the gold nanostructure. The second method is by indirect coupling, in which SH-linker B (part 1) can first be introduced on the gold nanostructure and then linker B (part 2)-prodrug can be added. The bond between linker B (part 1) and linker B (part 2) can be a triazole introduced by Huisgen click chemistry (See, Zhang et al Analytical Chemistry 2006 EST:7:1 pgs.A-H.) or a peptide bond by reaction of an amino group with a N-hydroxy succinimide (NHS).

In another embodiment the antigens used in the multivalent conjugate, as described herein, can be cancer antigens. The cancer antigens can be any cancer antigens that treat the specific cancers as described herein. For example, cancer cell antigens can include WT1,MUC1, LMP2, HPV E6, E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinase3, bcr-abl, Tyrosinase, Survivin, PSA, hTERT, Sarcoma translocation breakpoints, EphA2, PAP, ML-IAP, AFP, EpCAM, ERG, NA17, PAX3, ALK, androgen receptor, Cyclin B1, Polysialic acid, MYCN, RhoC, TRP-2, GD3, Fucosyl GM1, Mesothelin, PSCA, MAGE A1, sLe, CYP1 b1, PLAC1, GM3m, BORIS, Tn, MAGE-B2, DAM-10, SAGE, RAGE and the like.

There is at least one contrast agent, cytotoxic agent, prodrug and/or antigens coupled to the gold nanostructure (GNS) via a cleavable linker, but there can be 1 to 5, 2 to 3, 2 to 4, 2 to 5, 3 to 4, 3 to 5, 1 to 3 or 1 to 4 coupled contrast agents, cytotoxic agents, prodrugs and/or antigens.

The nanostructures can be in any shape and can include gold nanoparticles, gold nanoshells, gold nanocages, gold nanorods, and the like. In one aspect the nanostructures are gold nanorods, which have the advantage of large-scale synthesis, easy functionalization and colloidal stability.

The synthesis of gold nanostructures is well known in the art. Generally gold nanostructures are produced by liquid chemical methods by reduction of chloroauric acid, the solution being rapidly stirred while a reducing agent is added. As more and more of the gold atoms form, the solution becomes supersaturated and the gold starts to precipitate. Other methods can be used to produce the gold nanostructures, which include the Turkevich method, the Brust method, the Martin methods (I), (II) and (III) and the like. In one aspect the gold nanorods are synthesized according to the EI-Sayed's protocols. (B. Nikoobakht, M.A. EI-SAYED, Chem. Mater. 2003; 15, 1975.

The size of the nanostructures vary from 1 nm to 100 nm or from 1 nm to 150 nm or from 1 nm to 80 nm.

In another aspect the gold nanostructures and preferably nanorods. have present at their surface 1 to 100,000 amine groups or between 1,000 to 50,000 amine groups or between 1,000 to 9,000 amine groups or 6,000 to 9,000 amine groups.

A pharmaceutical composition comprising a first multivalent conjugate, as described herein, wherein T is a cytotoxic agent or a contrast agent and a second multivalent conjugate, as described herein, wherein T is a cancer antigen and a pharmaceutically acceptable vehicle.

In this pharmaceutical composition the cytotoxic agent is described herein, the contrast agent is described herein, and the cancer antigen is also described herein.

The pharmaceutically acceptable vehicle can be any acceptable carrier, adjuvant or vehicle that does not interfere with the pharmaceutical activity of the pharmaceutical composition and is not toxic to the host to which the pharmaceutical composition is administered. It includes solvents, dispersion media, coatings, absorption delaying agents and the like. These pharmaceutically acceptable vehicles are described in Remington's Pharmaceutical Sciences 21st edition 2005. An acceptable carrier can be, for example, saline, buffered saline and the like. It can be added to the pharmaceutical composition after its formulation.

The pharmaceutical compositions, as described herein, can also contain an additional therapeutic agent. This additional therapeutic agent can either be separately coupled to a different multivalent conjugate or administered without any coupling to the multivalent conjugate. It can be administered simultaneously or it can also be administered before or after the administration of the pharmaceutical composition, described herein. This additional therapeutic agent is selected from the group of an analgesic, an antimicrobial, an antibacterial, an antiviral, an antibiotic, an antiinflammatory, an antioxidant, an antihistamine, an antipruritic, an antipyretic, an additional anti-cancer agent and combinations thereof.

Adjuvants can also be administered with the pharmaceutical compositions that contain the second conjugate that has cancer antigens. Examples of adjuvants include alum, GM-CSF and the CpG oligodeoxynucleotides or a CpG-like oligodeoxynucleotides, Freund's incomplete adjuvant (IFA)m MF59®, poly(I:C), monophosphoryl lipid A (MPLA), TLR5 ligands such as bacterial flagellin, TLR7/8 ligands such as imiquimod, gardiquimod and R848, NOD2 ligands such as muramyl dipeptides and mixtures thereof.

The two multivalent conjugates in the pharmaceutical composition, described herein, can be administered together or at separate intervals; i.e., before, after or during the MRI or near-infrared fluorescence or cancer treatment, as described herein. In this embodiment the cancer cells can be imaged and then treated with the cytotoxic agents, as described herein or vice versa or the cancer cells imaged and then subjected to the cancer antigens, described herein, or vice versa or the cytotoxic agent is administered and then subjected to the cancer antigens, as described herein or vice versa. This can provide in some instances a double cancer therapy; i.e., when the first multivalent conjugate contains a cytotoxic agent and the second contains a cancer antigen.

In another embodiment the multivalent conjugate, as described herein, is used for distinguishing normal cells from abnormal cells. The normal cells and abnormal cells can be distinguished by a magnetic resonance imaging device or a near-infrared fluorescence imaging device.

In another aspect a method for distinguishing normal cells from abnormal cells is disclosed herein. This method comprises administering to a warm blooded animal the multivalent conjugate, as described herein, using a magnetic resonance imaging device or a near-infrared fluorescence imaging device for imaging the cells in said warm blooded animal and interpreting the images of the cells in the warm blooded animal and distinguishing normal cells from abnormal cells.

The abnormal cells that are being distinguished from normal cells are cancer cells. Various types of cancer cells that can be distinguished include can be administered topically, enterally, parentally, intravenously or orally and can treat cancers such as acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ,* embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ,* lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vaginal cancer and vulvar cancer.

A magnetic resonance imaging device (MRI), a near-infrared fluorescence, Positron emission tomography (PET) or Single photo emission computed tomography (SPECT) device is used in this to distinguish the normal cells from the abnormal cells. MRI and near-infrared fluorescence are well known imaging techniques. In MRI after administering the multivalent conjugate, as described herein, a constant magnetic field is applied to the warm blooded animal body causing average magnetic movement of many protons to become aligned with the direction of the magnetic static field. An electromagnetic wave at the resonant frequency is then applied to the body's tissue, thus producing radio frequency signals that are picked up by a receiver such as a coil. Additional spatially varying magnetic fields are applied at the same time to the body. The signals are specially characterized using the rapidly changing, local magnetic field and computer-processed to produce images of the body part of interest. MRI can produce axial images, sagital images and coronal images. Because the multivalent conjugate enters cancerous cells, it allows normal from abnormal tissues to be distinguished in the MRI image and can pinpoint cancerous cells in the body. The MRI can detect small tumors that might be cancerous. Because cancer cells have a resonance frequency different from healthy cells, they are readily identifiable.

In a particular aspect in this method, the magnetic imaging device operates with a magnetic field greater than 0.3 Tesla and preferably between 3 and 9.4 Telsa allowing a good contrast on MRI images.

Near-infrared fluorescence imaging is based on fluorescence, which is the property of certain molecules to absorb light at one wavelength and to emit light at another wavelength. Light in the near-fluorescence range has deeper tissue penetration and less absorption by hemoglobin and water than visible-range light. It also has low background noise due to less autofluorescence from surrounding tissues.

Near- infrared fluorescence imaging can be categorized into two types, which are fluorescence reflectance imaging (FRI) and tomographic fluorescence imaging. Both methods can be used in the present invention. It is preferable to use tomographic fluorescence imaging since it is a three dimensional imaging technique that can create tomographic images similar to MRI.

Positron emission tomography (PET) is a nuclear medical imaging technique that detects pairs of gamma rays emitted indirectly by a positron-emitting radionuclide, emitted into the body using the multivalent conjugate. It produces a three dimensional image.

Single-photon emission computed tomography (SPECT) is a nuclear medicine imaging technique using gamma rays and is able to provide 3D information. This technique is similar to PET, but gamma radiation is directly measures, whereas in PET positrons that annihilate with electrons up to a few millimeters away, cause two gamma photons to be emitted in opposite directions.

The contrast agents for near-infrared fluorescence imaging are described herein. The wavelength for excitation and emission depends on the fluorescence device utilized. See, Marshall et al The Open Surgical Oncology Journal 2010; 2, 12-25. Thus, the wavelength for excitation can vary from between 745 nm to 785 nm or from between 760 nm to 790 nm. The wavelength for emission can vary from between 800 nm to 835 nm or from between 820 nm to 830 nm.

The multivalent conjugate can be administered intravenously or intratumorally. Generally the multivalent conjugate is injected intravenously into the warm blooded animal body before or during the MRI, near-infrared fluorescence, PET or SPECT. The amount of multivalent conjugate that is injected depends on the type and location of the cells to be imaged. Generally 5 to 25 mls of multivalent conjugate are injected or 10 to 20 mls of multivalent conjugate are injected or 8 to 30 mls of multivalent conjugate are injected. They can be administered in a sole does or administered in several doses at specific times.

The multivalent conjugate, as described herein, for use in treating cancer cells is yet another embodiment of the present invention. This can be accomplished by photothermal therapy.

A method for treating cancerous cells is still yet another aspect of the present invention. This method comprises administering to a warm blooded animal the multivalent conjugate, as described herein, locating said cancerous cells in a warm blooded animal via images obtained from a magnetic resonance imaging device or a near-infrared fluorescence imaging device and heating said cancerous cells using near infrared laser irradiation. This type of therapy is called photothermal therapy and is a non-invasive or minimally invasive cancer treatment method that uses light energy to produce heat energy, which induces cancer cell death.

In photothermal therapy the tumors are heated to above 42° C but below 100°C to prevent carbonization. The temperatures can also range from 10°C to 65°C or from 45°C to 75°C or from 25°C to 95°C can also be used. The temperature will depend upon the type of cancer cell being subjected to photothermal therapy.

Usually they are heated for a period of between 3 and 10 minutes or between 6 to 12 minutes or between 1 to 30 minutes or between 1 to 15 minutes using a laser. The laser is a pulsed laser and the near infrared wavelength is between 750 nm to 900 nm or between 780 nm to 1 nm or between 750 nm to 1 nm.

The photothermal therapy methods of the present invention destroy the cancer cells. Also contemplated in this method is to administering the multivalent conjugate that contains at least one cytotoxic agent or cancer antigen in a separate B-subunit of Shiga toxin before, during or after the photothermal therapy.

The photothermal therapy may be performed using a continuous wave diode laser operating at the near- infrared wavelength of 800 nm or in a range between 750 nm to 900 nm with a maximum power of 1 W. For exposure times see the ICNIRP Guidelines in Health Physics 2000;79 (4):431-440, which is incorporated herein by reference.

The dose of the contrast agent used in MRI or near-infrared fluorescence procedures, as described herein will depend on the type of cancer and will vary with the location of the tumor in the warm blooded animal body. For example the contrast agent can be administered between 0.025 mmol/kg to 0.2 mmol/kg. It can also be administered between 0.05 mmol/kg to 0.1 mmol/kg or at 0.7 mmol/kg to 0.15 mmol/kg.

The multivalent conjugate, as described herein, for the preparation of a medicament to treat cancer cells is yet another aspect of the present invention.

Using the multivalent conjugate, as described herein, cancer cells can be treated or distinguished using one of the following:
(1) photothermal therapy,
(2) imaging using near-infrared fluorescence or MRI or Positron emission tomography (PET) or Single photo emission computed tomography (SPECT),
(3) photothermal therapy and administering cytotoxic agents or vice versa,
(4) imaging using near-infrared fluorescence or MRI and administering cytotoxic agents or vice versa,
(5) photothermal therapy and administering cancer antigens or vice versa, or
(6) imaging using near-infrared fluorescence or MRI and administering cancer antigens or vice versa.

The above embodiment permits treating the cancer cells with two different types of therapy in any order or first imaging the cancer cells then treating or first treating the cancer cells and then imaging.

In another aspect the multivalent conjugate having a cancer drug coupled to the B-subunit of Shiga toxin or a functional equivalent thereof, as described herein, or in any one of the methods, as described herein, or the pharmaceutical compositions, as described herein, will contain between 2 to 40 mg of multivalent conjugate/kilogram, which contains between .073 to 1.5 of the at least one cytotoxic agent or pharmaceutically acceptable salts thereof, or prodrug or antigen. In another aspect the multivalent conjugate, as described herein, will contain between 0.1 to 50 mg of multivalent conjugate/kilogram, which contains between .004 to 1.8 of the at least one cytotoxic agent or pharmaceutically acceptable salts thereof, or prodrug or antigen. In yet another aspect the multivalent conjugate, as described herein, will contain between 8 to 25 mg of conjugate/kilogram, which contains between 0.30 to 0.92 of the at least one cancer drug or pharmaceutically acceptable salta thereof, or prodrug or antigen.

The multivalent conjugate is administered intravenously or intratumorally, while the pharmaceutical composition or pharmaceutically acceptable salt thereof can be administered intravenously, orally, intratumorally, epidurally, intradermally, subcutaneously, intramuscularly and the like.

A number of embodiments and/or aspects of the invention have been described. Nevertheless it will be understood that various modifications may be made without departing from the spirit and scope of the invention.

### Materials and methods

All reactions were performed under argon atmosphere unless otherwise noted.

### Solvents:

THF was distilled from sodium and benzophenone, CH2Cl2 was distilled from P2O5. DMA, DMF and mesitylene were stored over molecular sieves. Cyclohexane and toluene were distilled. Other solvents were used without further purification.

### Analyses:

Thin layer chromatography analyses were performed over Merck 60 F254 sheets and visualized with UV light.

Flash chromatographies were carried out on silica gel 320-400 mesh. Yields refer to chromatographically and spectroscopically pure materials.
IR spectra were recorded with a Fourier transform infrared spectroscopy (PERKIN-ELMER 1710 spectrometer).

Melting points were determined for recrystallized compounds only, by capillary method and are uncorrected.

Positive and negative electrospray ionization spectra were performed on a WATERS ZQ 2000.

High resolution mass spectra were obtained at the Laboratoire de Spectrométrie de Masse of ICSN CNRS (Gif-sur-Yvette), or from Orleans.

1H NMR and 13C NMR spectra were recorded at room temperature with a BRUCKER ACP 300 at respectively 300 MHz and 75 MHz with complete proton decoupling. Chemical shifts are reported in ppm relative to the residual solvent peak as the internal reference, coupling constants J are given in Hertz. Spin multiplicities are given with the following abbreviations: s = singulet, d = doublet, dd = doublet of doublet, t = triplet, q = quadruplet, m = multiplet. Peak assignment was unambiguously performed using DEPT, HMQC and HMBC techniques.
Optical rotation was measured when required using a Perkin-Elmer 241 polarimeter.

### Examples

### Example 1-Synthesis of Tri-tert-butyl 2,2',2"-(1,4,7,10-tetraazacyclododecane-1,4,7-triyl)triacetate

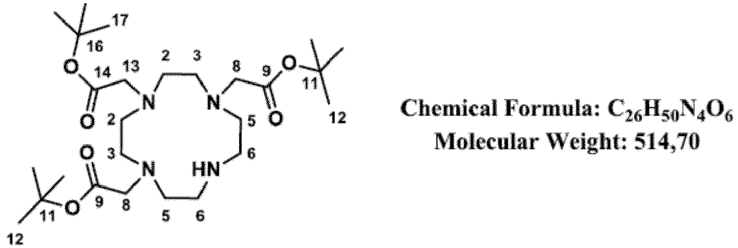

The protocol from O. Axelsson et al in WO200612723 2006 was used. Cyclen (1,4,7, 10-tetraazacyclododecane) (5.0 g, 29 mmol) and AcONa (7.22 g. 88 mmol) were introduced in dry DMA (80 mL) under argon. α-bromo-*tert*-butylacetate (13 mL, 87 mmol) dissolved in dry DMA (13 mL) was added drop by drop at 0 °C and the reaction was performed at room temperature during 5 days. At the end of the reaction, water (140 mL) was poured into the mixture and pH was adjusted at 9 with Na₂CO₃ before the introduction of KBr (5.05 g, 42.4 mmol). The mixture was filtrated and the beige precipitate was solubilized in CH2Cl2 and water was extracted. The organic layer was dried over Na2SO4, was concentrated, and the residue was washed with cooled EtOAc to isolate a white powder. This purification process led to the isolation of tri-*tert*-butyl-1,4,7,10-tetraazacyclododecane-1,4,7-triacetate as an amorphous white powder (10.9 g, 73 %).

**Mp** : 179 °C (recrystallised from toluene to give yellow crystals).
**Rf** : 0.7 (CH₂Cl₂/MeOH/NH4OH 80:15:5, Draggendorf visualization)
**1H NMR** (CDCl3, 300 MHz) δ 3.36 (s, 4H, **H8),** 3.28 (s, 2H, **H13),** 3.07 (m, 4H, **H6),** 2.88 (m, 12H, **H2, H3, H5),** 1.46 (s, 18H, **H12),** 1.45 (s, 9H, **H17). 13C NMR** (CDCl3, 75 MHz) δ 170.7 **(C9),** 169.9 **(C14),** 81,9 **(C16),** 81.7 **(C11**), 58.3 **(C8 - C13),** 51.5 **(C5),** 49.6 **(C2 - C3),** 47.7 **(C6),** 28.4 **(C17),** 28.3 **(C12).**
**MS** (ESI+) : *m*/*z* 515 [M+H]+.

### Example 2-Synthesis of Tri-tert-butyl 2,2',2"-(10-(2-(benzyloxy)-2-oxoethyl)-1,4,7,10 tetraazacyclododecane-1,4,7- triyl)triacetate

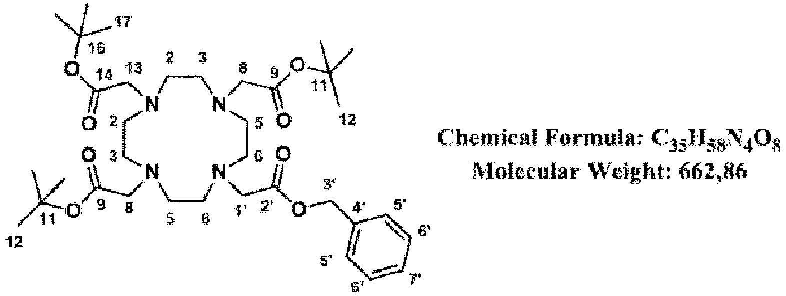

The procedure of Strauch, R. C., et al., J. Am. Chem. Soc. 2011, 133, 16346-49 was followed. More specifically, to a suspension of Tri-*tert-*butyl 2,2',2"-(1,4,7,10-tetraazacyclododecane-1,4,7-triyl)triacetate (5.10 g, 10 mmol) in acetonitrile, K2CO3 powder (4.14 g, 30 mmol, 3 eq.) and subsequently benzyl bromoacetate (3.43 g, 15 mmol) were added in acetonitrile 75 mL at 75 °C. Reaction was stirred at room temperature for 16 hrs. Reaction process was monitored by TLC. The precipitated solids were removed by filtration and the filtrate was concentrated to give the crude product, which was purified by silica gel column chromatography using CH₂Cl₂/MeOH (100:0 to 90:10) to give a colorless solid of Tri-*tert-*butyl 2,2',2"-(10-(2-(benzyloxy)-2-oxoethyl)-1,4,7,10 tetraazacyclododecane-1,4,7- triyl)triacetate (5.97 g, 90 %).

**Rf :** 0.8 (CHCl₃/MeOH 90:10).
**Mp:** 195 °C (recrystallized from toluene to give yellow crystals).
**1H NMR** (300 MHz, CDCl3) δ 7.33 (m, 5H, **H5', H6'** and **H7'),** 5.11 (s, 2H, **H3'),** 3.78 (s, 2H, **H1'),** 3.43 (br, 6H, **H8** and **H13),** 3.32-3.14 (m, 4H, **H6),** 3.04-2.88 (m, 12H, **H2, H3, H5),** 1.46 (s, 18H, **H12),** 1.45 (s, 9H, **H17).**
**13C NMR** (75MHz, CDCl3) δ 174.3 **(C9** and **C14),** 172.9 **(C2'),** 144.7 **(C4'),** 129.8 **(C6'),** 128.7 **(C7'),** 127.3 **(C5'),** 81.7 **(C11**), 81.5 **(C17),** 75.3 **(C3'),** 58.3, 57.1, 53.8 **(C8, C13, C1**'), 52.6, 52.3, 50.5 **(C2, C3, C4, C5),** 27.5 **(C17),** 27.4 **(C12).**
**MALDI** (*m*/*z*) for [M+H]+ calculated 662.8 found 663.4.
**MS (ESI+):** *m*/*z* 663.4 [M+H]+, 685.3 [M+Na]+.

### Example 3-Synthesis of 2-(4,7,10-Tris(2-(tert-butoxy)-2-oxoethyl)-1,4,7,10-tetraazacyclododecan-1-yl)acetic acid

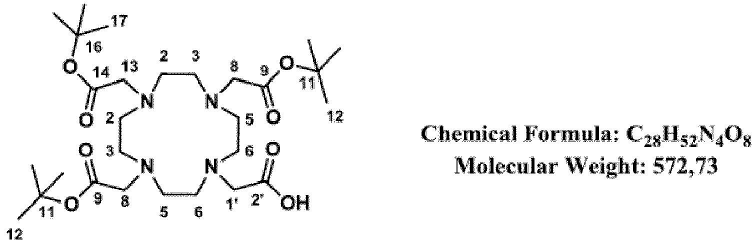

To a solution of tri-*tert*-butyl 2,2',2"-(10-(2-(benzyloxy)-2-oxoethyl)-1,4,7,10 tetraazacyclododecane-1,4,7- triyl)triacetate (2.65 g, 4 mmol) in 120 mL MeOH, palladium on carbon (10% Pd, 350.8 mg) was added and the reaction mixture was stirred under hydrogen atmosphere at room temperature for 4 hrs. The reaction was monitored by TLC (CH₂Cl₂-MeOH (1:1) (0.1% water), Rf = 0.6). After completion of the reaction, the catalyst was removed by filtration, the solvents were evaporated, and the crude mixture was rapidly purified by silica gel column chromatography (CH₂Cl₂ to CH₂Cl₂/MeOH/H2O, 90:10:0.1). The product was obtained as a colorless sol id of 2-(4,7,10-Tris(2-(*tert*-butoxy)-2-oxoethyl)-1,4,7,10-tetraazacyclododecan-1-yl)acetic acid (1.6 g, 70 %).
**Rf** : 0.2 (CH₂Cl₂/MeOH/H2O, 90:10:0.1).
**Mp :** 130 °C (recrystallised from Et2O to give colorless crystals).
**1H NMR** (300MHz, CD3OD) δ ) δ 3.85 (s, 2H, **H1'),** 3.54 (br, 6H, **H8, H13),** 3.38-3.30 (m, 4H, **H6),** 3.01-2.94 (m, 4H, **H5),** 2.86-2.71 (m, 8H, **H2, H3),** 1.46 (s, 18H, **H12),** 1.45 (s, 9H, **H17).**
**13C NMR** (75MHz, CD3OD) (d, ppm): 171.6 **(C2'),** 170.9 **(C9),** 169.7 **(C14),** 81.6 **(C16),** 81.3 **(C11),** 57.3 **(C1'),** 56.3 **(C8),** 55.6 **(C13),** 53.2-50.9 **(C2, C3),** 50.5-47.7 **(C5, C6),** 27.5 **(C17),** 27.4 **(C12).**
**MS** (ESI) : *m*/*z* 571 [M-H]-, 597 [M+Na]+.

### Example 4- Synthesis of tri-tert-butyl 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetate

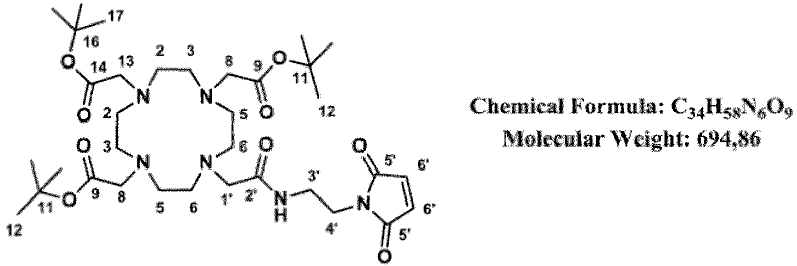

To a solution of product 2-(4,7,10-Tris(2-(*tert*-butoxy)-2-oxoethyl)-1,4,7,10-tetraazacyclododecan-1-yl)acetic acid (572.7 mg, 1 mmol) in dry DMF (15 mL), amino-maleimide **137** (483.3 mg, 1 mmol) was added with HATU (272 mg, 1.4 mmol) and DIPEA (360 µL, 1.84 mmol). The mixture was stirred overnight at room temperature. After removing the solvent under the vacuum, the crude product was purified by flash chromatography on silica gel (CH₂Cl₂/MeOH, 90:10) to give compound tri-*tert*-butyl 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetate as a white foam (396 mg, 57 %).
**Rf :** 0.3 (CH₂Cl₂/MeOH, 90:10).
**1H NMR** (300MHz, CDCl3) δ 8.30 (b, 1H, **NH),** 6.86 (s,2H, **H6'),** 3.78-3.54 (br, 4H, **H3', H4'),** 3.54 (br, 4H, **H13, H1'),** 3.48 (br, 4H, **H8),** 3.09-2.99 (m, 8H, **H2, H3),** 2.97-2.86 (m, 8H, **H5, H6),** 1.46 (s, 18H, **H12),** 1.45 (s, 9H, **H17).**
**13C NMR** (75MHz, CDCl3) δ 171.6 **(C2'),** 171.0 **(C5'),** 170.4 **(C9, C14),** 134.2 **(C6'),** 81.5 **(C16),** 81.3 **(C11),** 57.8 **(C1'),** 55.7-55.2 **(C8, C13),** 54.1-51.5 **(C2, C3),** 51.0-48.9 **(C5, C6),** 38.0 **(C4'),** 32.7 **(C3'),** 27.8 **(C17),** 27.6 **(C12).**
**MS** (ESI) : *m*/*z* 695 [M + H]+.

### Example 5- Synthesis of 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetic acid

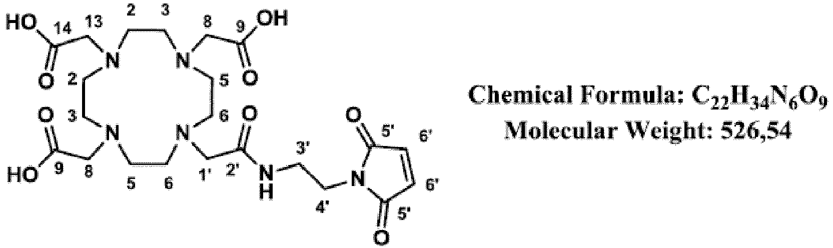

The procedure described by Barge, A., et. al., Org. Biomol. Chem., 2008, 6, 1176-84 was used. More specifically tri-*tert*-butyl 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetate (200 mg, 0.29 mmol) was dissolved in 6 ml of CH₂Cl₂:TFA acid (1:2). This solution was stirred at room temperature for about 9 hr. After removing the solvent, the residue was dissolved in 1 mL hot methanol. Diethyl ether (10 mL) was added and the final off-white solid product of 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetic acid (140.5 mg, 92 %) was obtained.
**Rf : 0.3** (CH₂Cl₂/MeOH, 90:10).
**Mp** : > 250 °C.
**1H NMR** (300MHz, CD3OD) δ 8.30 (b, 1H, **NH),** 6.86 (s,2H, **H6'),** 3.78-3.54 (br, 4H, **H3', H4'),** 3.63 (br, 6H, **H8, H13),** 3.54 (br, 4H, **H13, H1'),** 3.32-3.15 (m, 8H, **H2, H3),** 3.09-3.01 (m, 8H, **H5, H6),** 1.48 (s, 18H, **H12),** 1.47 (s, 9H, **H17).**
**13C NMR** (75MHz, CD3OD) δ 173.2 **(C13, C9),** 173.0 **(C2'),** 172.6 **(C5'),** 135.1 **(C6'),** 56.7-55.2 **(C8, C13),** 55.0 **(C1'),** 53.1-51.2 **(C2, C3),** 49.9-48.6 **(C5, C6),** 38.0 **(C4'),** 32.7 **(C3').**
**MS** (ESI) : *m*/*z* 527 [M + H]+.

### Example 6- Synthesis of DOTA-Gd-maleimide

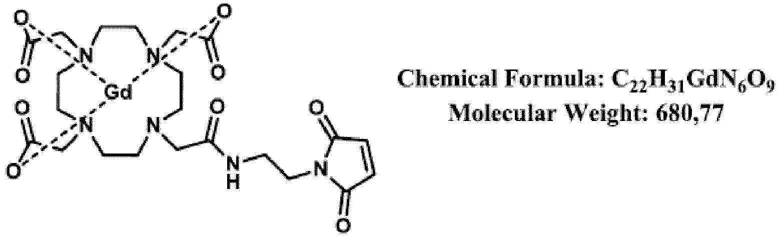

To a solution of 2,2',2"-(10-(2-((2-(2,5-dioxo-2,5-dihydro-1H-pyrrol-1-yl)ethyl)amino)-2-oxoethyl)-1,4,7,10-tetraazacyclododecane-1,4,7-triyl)triacetic acid **139** (100 mg, 0.19 mmol) in H2O (5 ml) was slowly added GdCl₃·6H₂O (70.5 mg, 0.19 mmol) maintaining pH 5 by addition of NaOH (0.1 M). The solution was stirred for 4 h at room temperature and filtered through Millipore® (HA 0.22 µm). The solution was concentrated and the residue was purified on Amberlite® XAD 1600 resin. Complex DOTA-Gd-maleimide was obtained as a white solid (119 mg, 92 %).
**Mp :** > 250°C.
**MALDI TOF MS:** (ESI) *m*/*z* 618 [M+H]+.

### Example 7-Synthesis of tri-tert-butyl 2,2',2"-(10-(2-((2,5-dioxopyrrolidin-1-yl)oxy)-2-oxoethyl)-1,4,7,10-tetraazacyclododecane-1,4,7-triyl)triacetate

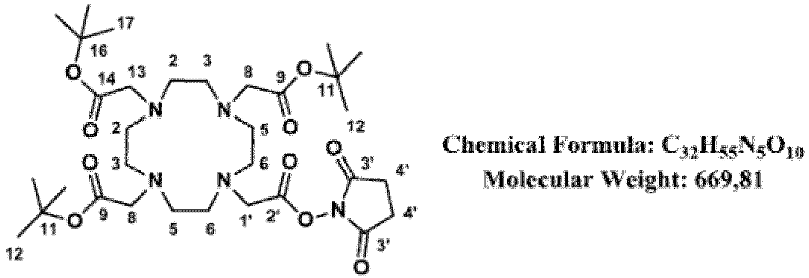

The procedure that was followed was described in Li, C., et al., J. Am. Chem. Soc., 2006, 128, 15072-73. Compound 2-(4,7,10-Tris(2-(*tert-*butoxy)-2-oxoethyl)-1,4,7,10-tetraazacyclododecan-1-yl)acetic acid (400 mg, 0.70 mmol), *N*-hydroxysuccinimide (90.4 mg, 0.78 mmol, 1.1 equiv.), and Obenzotriazol- 1-yl-*N,N,N',N*'-tetramethyluronium hexafluorophosphate (HBTU) (296 mg, 0.78 mmol, 1.1 equiv.) were dissolved in 10 mL of acetonitrile. The reaction was stirred at room temperature for 24 hr. After removing the solvent under vaccum, the crude product was purified by flash chromatography on silica gel (CH₂Cl₂/MeOH, 85:15) to give compound tri-*tert*-butyl 2,2',2"-(10-(2-((2,5-dioxopyrrolidin-1-yl)oxy)-2-oxoethyl)-1,4,7,10-tetraazacyclododecane-1,4,7-triyl)triacetate as a white foam (404 mg, 86 %).
**Rf :** 0.3 (CH₂Cl₂/MeOH, 85:15).
**1H NMR** (300MHz, CDCl3) δ 3.51 (br, 4H, **H8),** 3.54 (br, 4H, **H13, H1'),** 3.32-3.26 (m, 4H, **H6),** 3.08-2.99 (m, 4H, **H5),** 2.97-2.86 (m, 8H, **H2, H3),** 2.85 (s, **H4'),** 1.46 (s, 18H, **H12),** 1.45 (s, 9H, **H17).**
**13C NMR** (75MHz, CDCl3) δ 173.4 **(C9, C14),** 173.1 **(C2'),** 169.9 **(C3'),** 82.6 **(C16),** 82.4 **(C11),** 55.8 **(C8),** 55.7 **(C13),** 54.2 **(C1'),** 54.1-51.5 **(C2, C3),** 51.0-48.9 **(C5, C6),** 27.8 **(C17),** 27.6 **(C12).** 25.6 **(C4')**
**MS** (Cl/NH3) *m*/*z* 692 [M + H]+

### Example 8- Synthesis of 2,2',2"-(10-(2-((2,5-dioxopyrrolidin-1-yl)oxy)-2-oxoethyl)-1,4,7,10-tetraazacyclododecane-1,4,7-triyl)triacetic acid

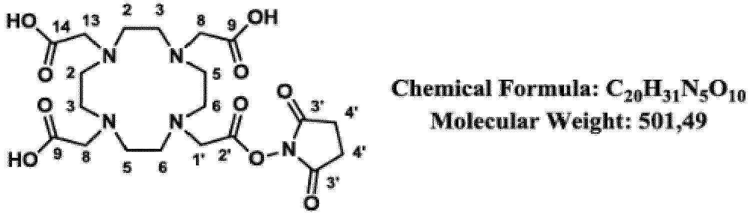

Compound tri-*tert*-butyl 2,2',2"-(10-(2-((2,5-dioxopyrrolidin-1-yl)oxy)-2-oxoethyl)-1,4,7,10-tetraazacyclododecane-1,4,7-triyl)triacetate(400 mg, 0.6 mmol) was dissolved in 9 ml of CH₂Cl₂:TFA (1:2). This solution was stirred at room temperature for about 6 h. After removing the solvent, the residue was dissolved in 1 mL hot methanol. Diethyl ether (20 mL) was added and the final white solid product 2,2',2"-(10-(2-((2,5-dioxopyrrolidin-1-yl)oxy)-2-oxoethyl)-1,4,7,10-tetraazacyclododecane-1,4,7-triyl)triacetic acid (276 mg, 92%) was obtained.
**Rf :** 0.0 (CH₂Cl₂/MeOH, 1:1).
**1H NMR** (300MHz, CD3OD) δ 4.09 (br, 6H, **H8, H13),** 3.54 (br, 2H, **H1'),** 3.32-3.12 (m, 12H, **H2, H3, H5),** 3.12-2.98 (m, 4H, **H6),** 2.84 (s, **H4').**
**13C NMR** (75MHz, CD3OD) δ 175.4 **(C9, C14),** 173.8 **(C2'),** 171.5 **(C3'),** 55.8-55.6 **(C8, C13, C1**'), 55.7-54.1 **(C2, C3),** 53.8-52.3 **(C5, C6),** 25.6 **(C4').**
**MS** (IC/NH3) : *m*/*z* 502 [M+H]+.

### Example 9-Preparation of DOTA conjugated gold nanorods (GNR)

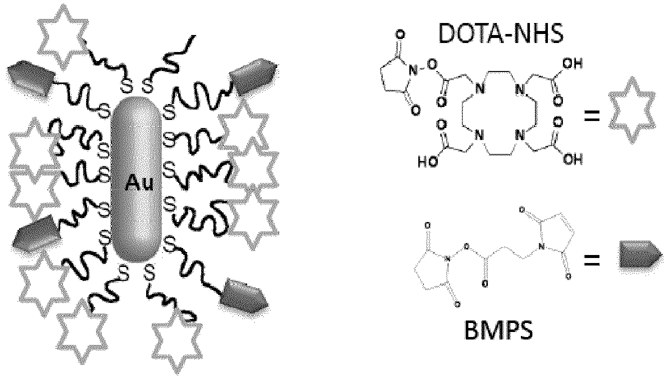

To the solution of compound gold narorods GNR-S-PEG5000NH2 (25 nM, based on gold nanorods) in 2 mL of pH 7.5 PBS buffer, 20 µL of DOTA-NHS ester (1.9 mM {1 mg/1.05 mL DMSO}, 7600 eq.) was added and 20 µL of 2,5-dioxopyrrolidin-1-yl 3-(2,5-dioxo-2,5-dihydro-1H-pyrrol-1-yl)propanoate BMPS (38 µM {1 mg/98.8 µL DMSO}, 200 eq. The mixture was stirred at room temperature for 1 hour and the resulting product was purified by chromatography with a PBS equilibrated Sephadex G-100 column and eluted with PBS and was passed through a column filled with 4 ml of Sephadex G-25 gel (equilibrated with PBS). Elution was performed with PBS (14 ml) under a slight air pressure (to increase flow rate), to afford the title product of DOTA conjugated GNR.
**IR** : 2981 (carboxylic acids band) {amine band disappeared}.

### Example 10-Synthesis of GNR-DOTA-Gd-STxB

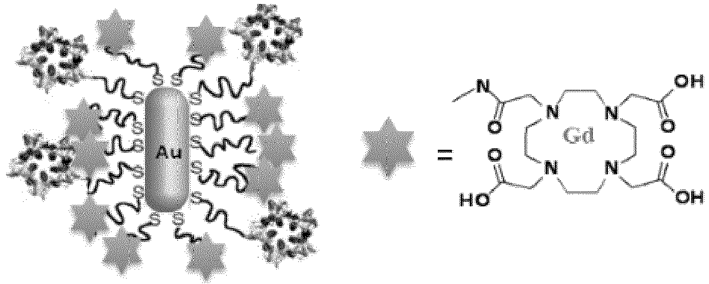

To the solution of compound functionalized gold narorods (190 µM, based on gold DOTA and 3.8 µM based on maleimide) in 2 mL PBS buffer pH 7.5, 20 µL of GdCl₃·6H₂O (1.9 mM {1 mg/1.4 mL DMSO}, 1 eq.) was added and 20 µL of STxB (38 µM in PBS). The mixture was stirred at room temperature for 4 hours and the resulting product was passed through a column filled with 5 ml of Sephadex G-100 gel (equilibrated with PBS). Elution was performed with PBS (10 ml) under a slight air pressure (to increase flow rate), to afford the title product GNR-DOTA-Gd-STxB. The weight percentages of Gd on STxB-GNR-DOTA-Gd were calculated..
IR : carboxylic acids band disappeared due to the complexation with gadolinium.

### Example 11 -Protocol for GNR-S-PEG₅₀₀₀-NH₂ functionalization with N-[β-Maleimidopropyloxy] succinimide ester

In low surface coverage degree (1% of the GNR surface is functionalized with SH-PEG-NH₂, the rest of the GNR surface is stabilized by unreactive SH-PEG-OMe) the amine terminus of PEGylated GNR are reacted with a molecular linker, N-[β-Maleimidopropyloxy] succinimide ester (BMPS) before the biomolecule is coupled.

Having created a functional amino group at the surface of the GNRs this can be used to introduce a further linker, N-[β-Maleimidopropyloxy] succinimide ester (BMPS; Alfa Aesar). This Maleimido- compound enables to link in the next step covalently GNR to STxB. 1 ml GNR-S-PEG₅₀₀₀-NH₂ in PBS (OD 4.5) was treated with 20 µl BMPS (10mM in DMSO), vigorously shaken and left to react for 1 hour. The mixture was purified by chromatography using a PBS equilibrated Sephadex G-100 filled column and eluted with PBS, yielding 1 ml GNR-S-PEG₅₀₀₀-NH-Mal (OD 4). BMPS was used right after preparation and kept in the freezer while unused.

### Example 12-Conjugation of GNR-S-PEG₅₀₀₀-NH-Mal to RGD-Cys

Freshly prepared GNR-S-PEG₅₀₀₀-NH-Mal (1 ml, OD 4) was treated with 22 µl STxB (100 µM in PBS, ≈ 10 eq. / NH-Mal), vigorously shaken and left to react for 3 hours at room temperature. The mixture was purified by 4 centrifugation steps at rcf of 8000 for 40 minutes, followed by supernatant replacement with PBS, yielding 1 ml GNR-S-PEG₅₀₀₀-NH-Mal-STxB (OD 4).

Measuring IR spectra of the compounds revealed a successful functionalization of the GNR with STxB. The IR spectra of GNR-S-PEG₅₀₀₀-NH-Mal-STxB, showed bands characteristic for PEG, *i.e*. strong C-H and C-O stretching around 2861 and 1100 cm⁻¹, respectively. Additionally characteristic amine bands of STxB at about 3300, 1650 and 1550 cm⁻¹) was observed.

### Example 13- Protocol for GNR-S-PEG₅₀₀₀-NH₂ functionalization with BMPS (N-[β-Maleimidopropyloxy] succinimide ester) and DOTA-NHS (1,4,7,10-tetraazacyclododecane-N,N',N",N"'-tetraacetic acid mono-(N-hydroxysuccinimidyl) ester

Using amine PEGylated GNR 1,4,7,10-tetraazacyclododecane-N,N',N",N"'-tetraacetic acid mono-(N-hydroxysuccinimidyl) ester (DOTA-NHS) was linked. Creating anchor groups for STxB at the same time, DOTA-NHS was mixed with 3-Maleimidopropionic acid N-hydroxysuccinimide ester (BMPS) in a mol. Ratio of 98 : 2 (DOTA-NHS : BMPS) before Gd and STxB were allowed to react at the surface of the GNR. Briefly the reaction was carried out under following conditions. To 2 ml GNR-S-PEG₅₀₀₀-NH₂ (λ=744 nm, OD=2; 4 nM) 13.8 µl of a 8mM mixture of DOTA-NHS/BMPS in DMSO (98:2; 10x excess) was added. At room temperature. the mixture was slightly mixed during 1 hour. After purification from excess of reactant using four cycles of centrifugation (rcf 8000, 40 minutes) and supernatant replacement by PBS, the functionalized GNR pellet was finally re-suspended in fresh PBS, yielding 2 ml GNR-S-PEG₅₀₀₀-2%NH-Mal-98%DOTA (OD2, λ=746 nm; 4nM).

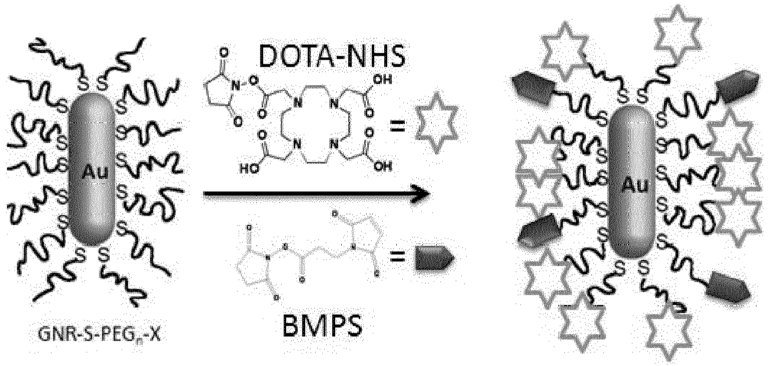

The above schematic illustrates surface functionalization of GNR-PEG-NH₂ with BMPS and DOTA-NHS.

### Example 14-Protocol for GNR-S-PEG₅₀₀₀-2%NH-Mal-98%DOTA functionalization with STxB

Then 84 µL STxB-Cysteine (10 µM in PBS, ≈ 1 eq. / NH-Mal), solution in PBS was added to the fresh prepared GNR-S-PEG₅₀₀₀-2%NH-Mal-98%DOTA (2ml, OD 2, λ=746 nm). Purification was performed using four cycles of centrifugation (rcf 8000, 40minutes each cycle), yielding 2 ml GNR-S-PEG₅₀₀₀-2%NH-Mal-98%DOTA (OD 2, λ=749 nm, 4 nM).

### Example 15-Protocol for GNR-S-PEG₅₀₀₀-2%NH-Mal-STxB-98%DOTA functionalization with Gd³⁺

Afterwards 13µL GdCl₃ (8 mM in PBS, ≈ 5 eq. / NH-DOTA), was finally added to the colloidal dispersion and left for reaction at room tempaerature for 45 minutes. Finally the Gd incorporated GNR were dialyzed 3 times against fresh PBS (1 L bath, changed three times within two days) to get rid of the toxic free GdCl₃ or eventual Gd³⁺ in the solution.

2 ml of GNR-S-PEG₅₀₀₀-2%NH-Mal-98%DOTA-Gd (OD 2, λ +744 nm) could be collected after purification.

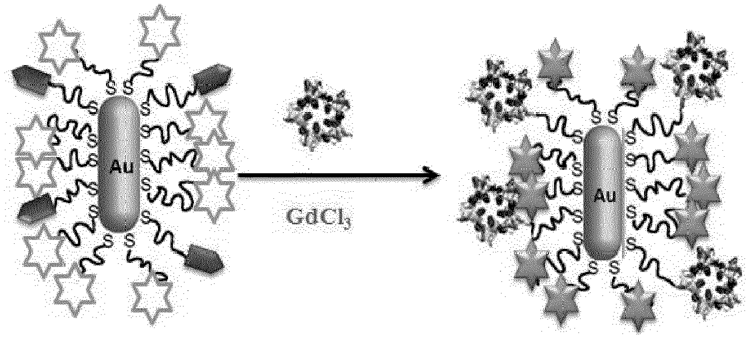

The above illustrates the schematic surface functionalization of GNR-PEG-NH-DOTA/BMPS with STxB and Gd³⁺.

### Example 16- Internalization of the conjugate STxB-(DOTA-Gd)₅ & tests of cellular internalization

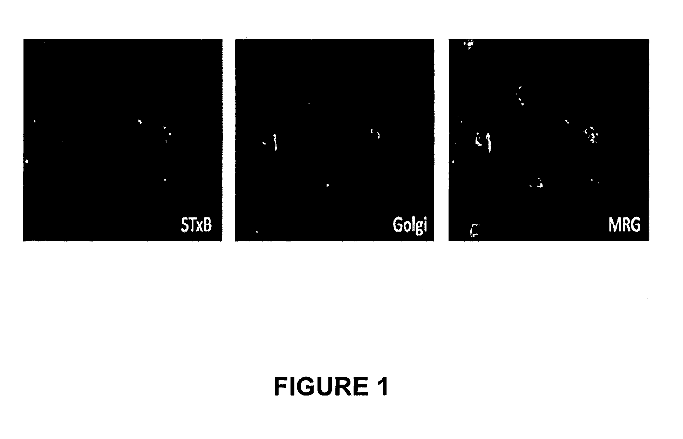

24h before contact, HeLa cells were seeded on glass slides at a final density of 70000 cells/well in DMEM supplemented with 10% FCS. Binding of STxB-(DOTA-Gd)₅ cleavable conjugates to HeLa cells was performed at 4°C for 30 min at a final concentration of 0.2 µM (STxB monomer); cells were then washed twice in complete medium and incubated for 40 min at 37°C. The intracellular localization of STxB-(DOTA-Gd)₅ conjugates was characterized by immunofluorescence detection after cell fixation in 4% PFA. Retrograde transport of the conjugate was confirmed by co-localization of STxB with Golgi markers, observed by epifluorescence microscopy as shown in Figure 1.

### Example 17-Evaluation of the Stability of the multivalent Conjugate

HSC-3 carcinoma cells are cultured in DMEM supplemented with 10% v/v heat-inactivated fetal bovine serum, 2 mM L-glutamine, penicillin and streptomycin in a 5% CO₂ atmosphere. Female mice, 7-8 weeks of age are injected subcutaneously in the flack with 100 µl of HSC-3 cells suspended in PBS. Once the tumor reached 3 mm in diameter, the mice are anesthesized with ketamine/xylazine/acepromazine and 100 microliters of the gold nanorods from Example 10 (GNR-DOTA-Gd-STxB) are injected intravenously. The nanorods circulated 24 hours prior to near infrared exposure. (1.7-1.9 W/cm² 6 mm dia., 10 minutes) Tumor volume is calculated as V=(d)²(D)(n/6).

### Example 18-Preparation of GNR 5%[-S-PEG₅₀₀₀-CF680]-2%[-S-PEG₅₀₀₀-NH₂]

A mixture of commercial SH-PEG₅₀₀₀-OMe (16.8mM), SH-PEG₅₀₀₀-NH₂ (16.8mM) and SH-PEG₅₀₀₀-NH-CF680 (16.8mM) compound was prepared in a volume ratio of 93 : 2 : 5. Subsequently 20 µl of 16.8 mM HS-PEG₅₀₀₀-X solution were added to 2 ml of GNR-CTAB (OD 2; c(GNR) = 27.8nM). The solution was mixed by vortex, covered with aluminum foil and left undisturbed at room temperature for 24 hours.

Purification from unreacted PEG compound and liberated CTAB was performed with the help of a Sephadex G-100, PBS equilibrated, filled column. The functionalized GNR 5%[-S-PEG₅₀₀₀-CF680]-2%[-S-PEG₅₀₀₀-NH₂] (2 ml, OD 2) was eluted with PBS.

### Example 19-Protocol for GNR 5%[-S-PEG₅₀₀₀-CF680]-2%[-S-PEG₅₀₀₀-NH₂] functionalization with N-[β-Maleimidopropyloxy] succinimide ester

Having created a functional amino groups at the surface of the GNRs they are used to induce N-[β-Maleimidopropyloxy] succinimide ester (BMPS; Alfa Aesar), which allows in the following step of the reaction to link covalently GNR to STxB. BMPS was used right after preparation and keep it in the freezer while unused. 2 ml GNR-5%[-S-PEG₅₀₀₀-CF680]-2%[-S-PEG₅₀₀₀-NH₂] in PBS (OD 2) was treated with 43.6 µl BMPS (100 □M in DMSO; 10eq / NH₂), vigorously shaken and left to react for 1 hour. The mixture was purified by chromatography using a PBS equilibrated Sephadex G-100 filled column and eluted with PBS, yielding 2 ml GNR-5%[-S-PEG₅₀₀₀-CF680]-2%[-S-PEG₅₀₀₀-NH-Mal] (OD 2).

### Example 20-Conjugation of GNR 5%[-S-PEG₅₀₀₀-CF680]-2%[-S-PEG₅₀₀₀-NH-Mal] to STxB-Cys

Freshly prepared GNR 5%[-S-PEG₅₀₀₀-CF680]-2%[-S-PEG₅₀₀₀-NH-Mal] (2 ml, OD 2) was treated with 43.6 µl STxB (100 µM in PBS, ≈ 10 eq. / NH-Mal), vigorously shaken and left to react for 3 hours at room temperature. The mixture was purified by 4 centrifugation steps at rcf of 8000 for 40 minutes, followed by supernatant replacement with PBS, yielding 2 ml GNR-5%[-S-PEG₅₀₀₀-CF680]-2%[-S-PEG₅₀₀₀-NH-Mal-STxB] (OD 2).

The IR spectra of GNR-S-PEG₅₀₀₀-NH-Mal-STxB, showed bands characteristic for PEG, *i.e*. strong C-H and C-O stretching around 2861 and 1100 cm⁻¹, respectively. Additionally characteristic amine bands of STxB at about 3300, 1650 and 1550 cm⁻¹ could be observed.

### Example 21- Evaluation of GNR-CF680-STxB conjugate biodistribution

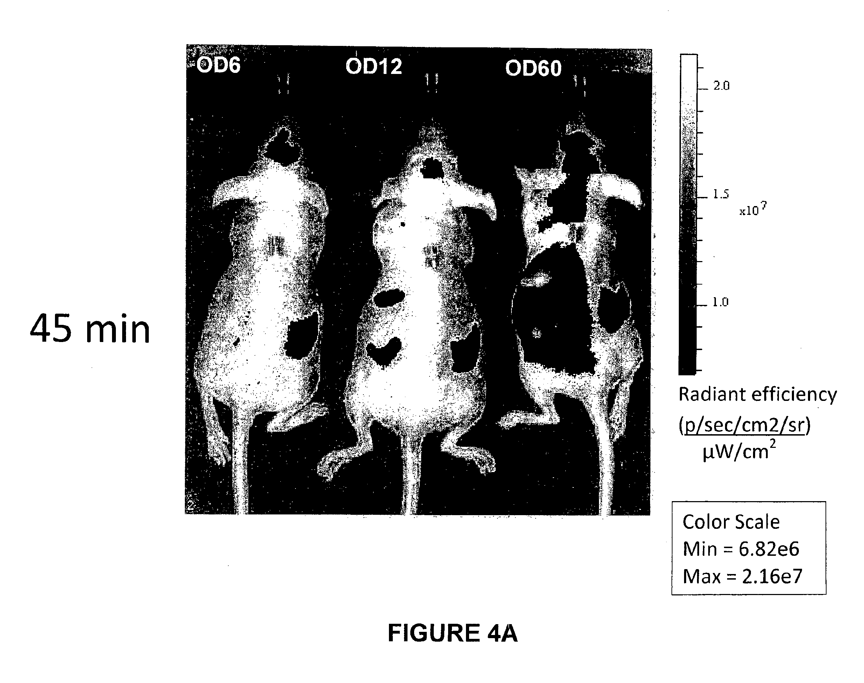

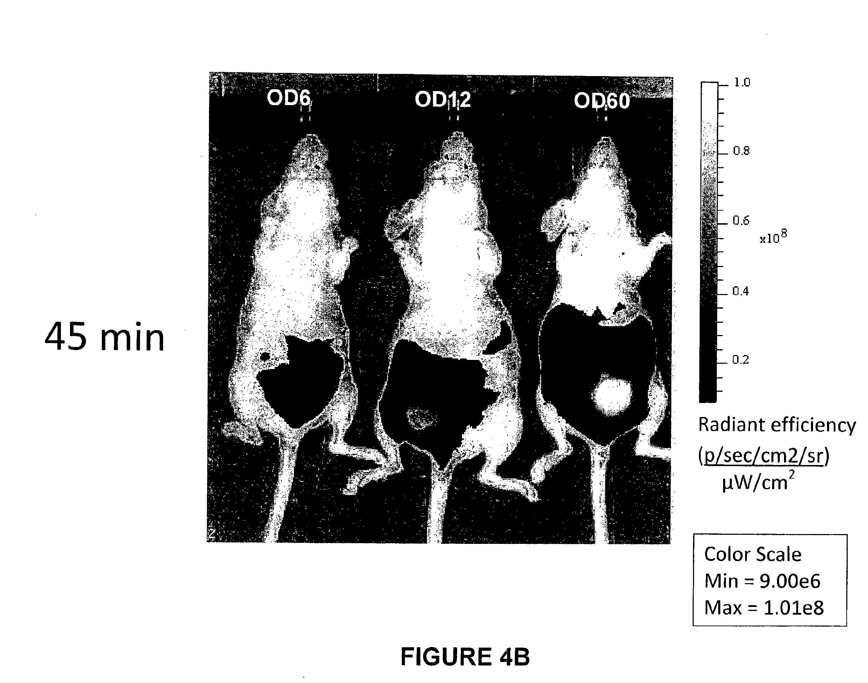

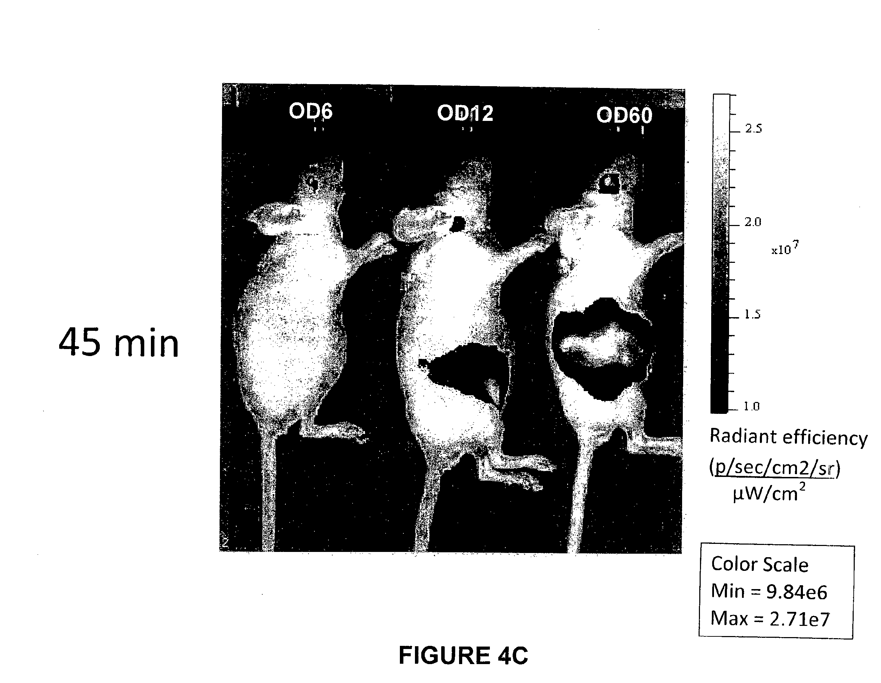

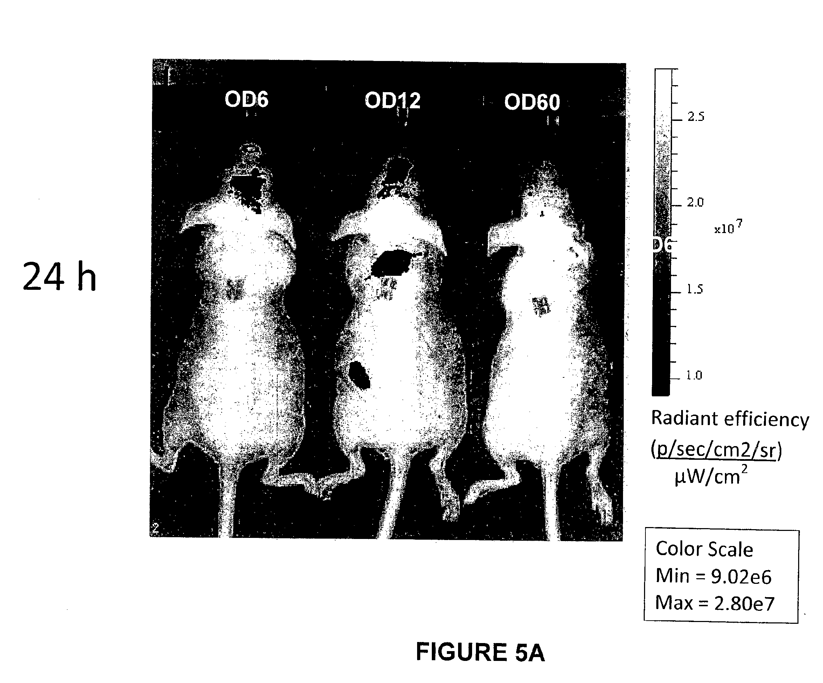

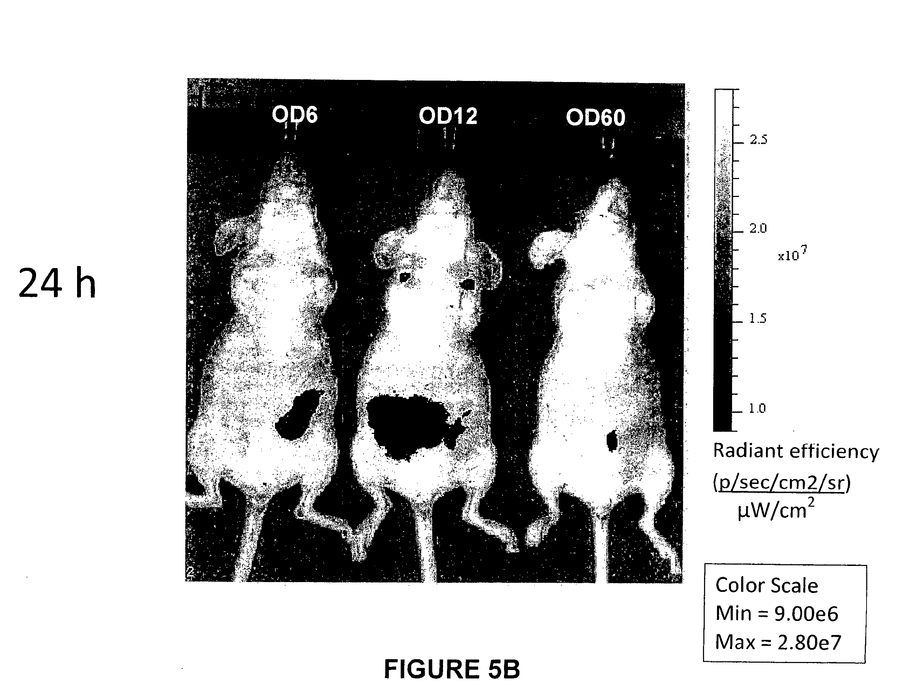

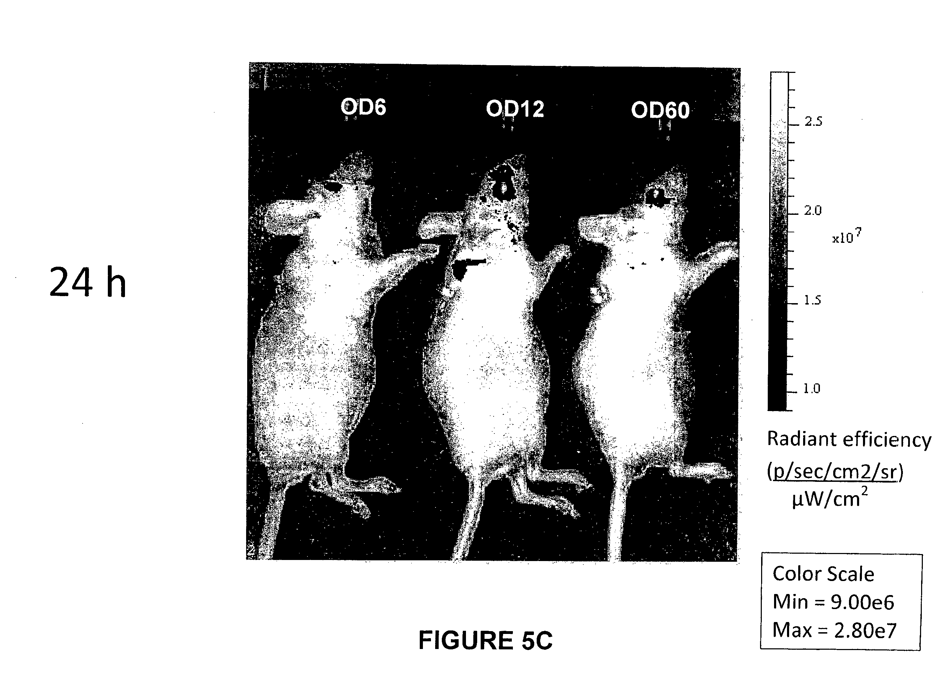

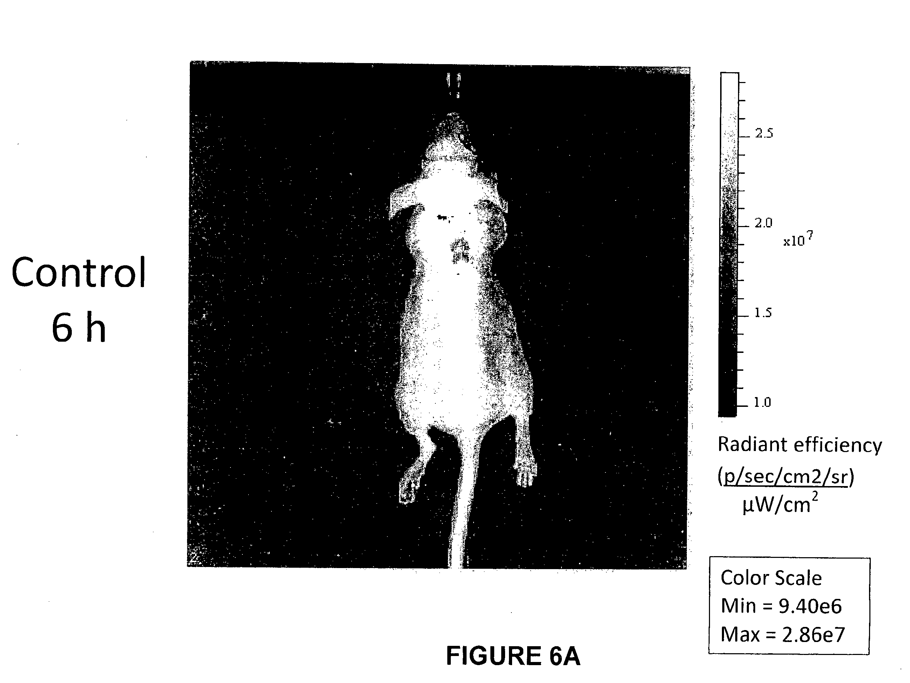

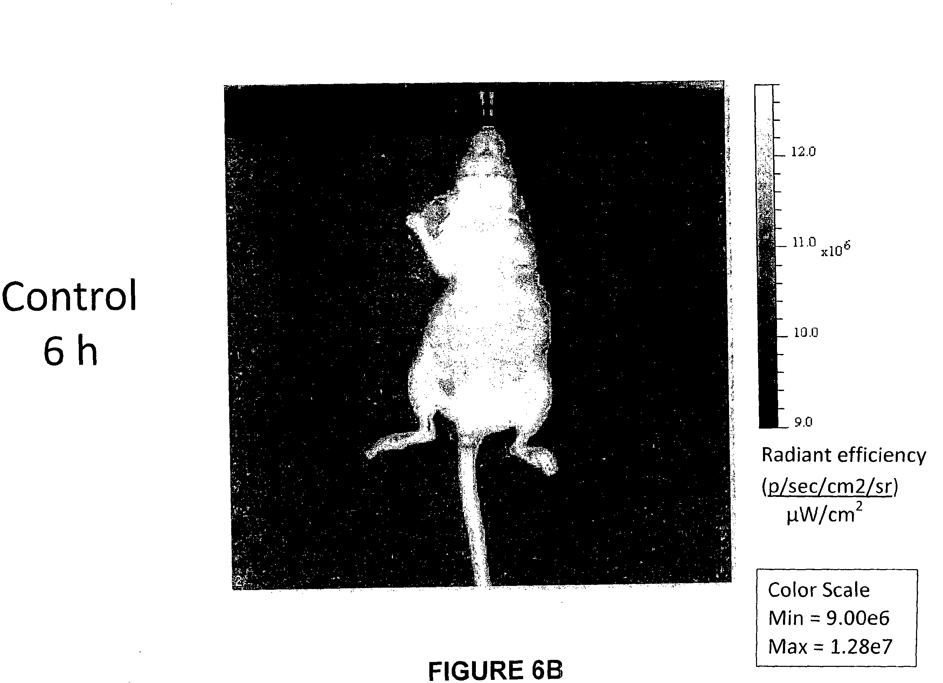

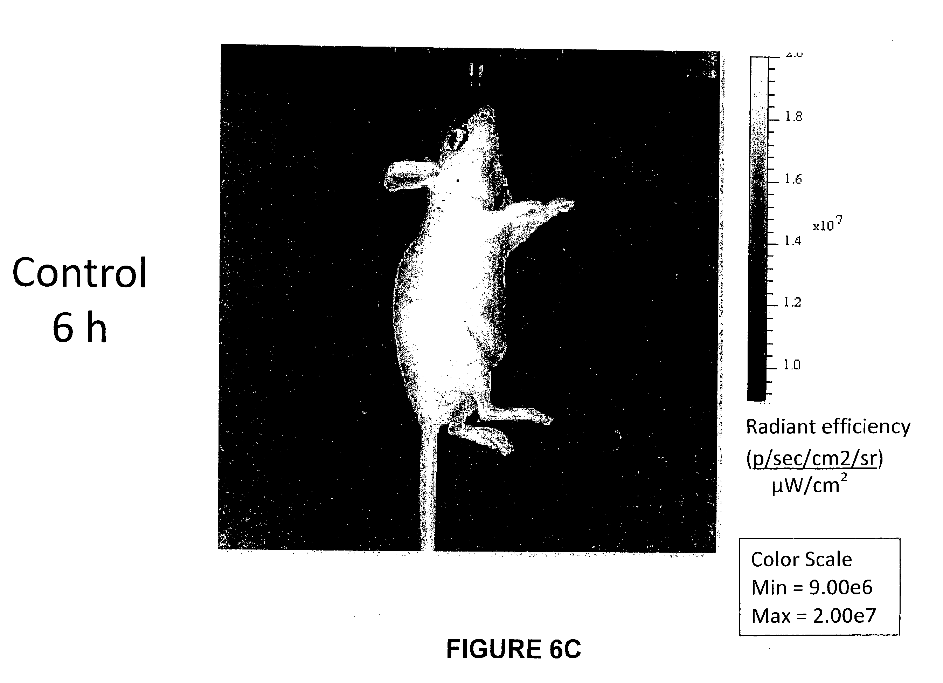

13-weeks-old female nude mice were subcutaneously xenografted in the flank with BC52 (EDWp14) human breast carcinoma cells. 8 weeks later (tumor around 5 mm in diameter), mice were anesthesized with ketamine/xylazine/acepromazine, and 100 microliters of the gold nanorods GNR-CF680-STxB were injected intraperitoneally, at different optical densities (different GNR concentrations), from 0 to 60. Whole animal imaging by near infrared fluorescence was performed at different time points after injection. After 1h, large amounts of GNR were eliminated through the kidney and bladder. 24h after injection, GNR-CF680-STxB clearly accumulated in tumor (Figure 4). *Ex-vivo* imaging of organs confirmed a dose-dependent accumulation of GNR conjugates in tumor tissue.

A number of embodiments and/or aspects of the invention have been described. Nevertheless it will be understood that various modifications may be made without departing from the spirit and scope of the invention.

## Claims

1. A multivalent conjugate comprising the following formula:
(STxB-linker A-S)ₓ-GNS-(S-linker B-T)_{y}
wherein STxB is the B-subunit of Shiga toxin; linker A is a noncleavable linker; linker B is a cleavable linker used to release at least one T or a noncleavable linker; GNS is a gold nanostructure; S is a sulfhydryl group and x and y can vary from 1 to 10,000; and T is a molecule selected from the group of: contrast agents, cytotoxic agents, prodrugs and antigens.

2. The multivalent conjugate according to Claim 1, wherein the noncleavable linker A is selected from the group comprising polyethylene glycols, polyacylamides, polysaccharides, methyl methacrylates, polyvinylalcohols, polyvinylpyrrolidones , polystyrenes, polysulfobetaines and mixtures thereof.

3. The multivalent conjugate according to any one of Claim 1 or Claim 2, wherein the cleavable linker B is selected from the group comprising 3-maleimidobenzoic acid N-hydroxyysuccinimide ester (MBS), N-succinimidyl 3-[2-pyridyldithio]-propionate (SPDP), [S-(N-succinimidyl)thioacetate] (SATA), [(N-succinimidiyloxy carbonyl)1-methyl-1-(2-pyridyldithio) toluene] (SMPT), 2-iminothiolane (2-IT), (Sulfosuccinimidyl N-[3-(Acetylthio)-3-methylbutyryl)-beta-alanine]) (sulfoNHS-ATMBA), thioimidates such as AMPT and M-CDPT and 2-[N-chlorocarbonyl)-N-methylamino]1-ethyl 2-pyridyldisulfide and a maleimide linker.

4. The multivalent conjugate according to any one of Claims 1 to 3, wherein the contrast agent is selected from the group comprising indocyanine green (IcG), 1,1'-bis-(4-sulfobutyl) indotricarbocyanine-5,5'-dicarboxylic acid diglucamide monosodium salt (SIDAG).disulfated indocyanine, tetra-sulfated indocyanine, y cyanine dyes, Cy7, Cy5.5, XenoLight CF680, Xenolight CF 750, Xenolight CF 770, AlexaFluor^{®} 647, AlexaFluor^{®} 680, AlexaFluor^{®} 700, AlexaFluor^{®} 750, DyLight 650, DyLight 680, DyLight 750, DyLight 755, heptamethine cyanine, positron emitting radioisotopes and gamma-emitting radioisotopes and mixtures thereof,
or the cytotoxic agent is selected from the group comprising halichomdrin B, maytansine, rhizoxin, taxanes as taxol and taxotere, taxol derivatives, vinca alkaloids as vinblastine and vincristine, ecteinascidin, bryostatin, eleutherobin, gemcitabine, podophyllotoxins, podophyllotoxins derivatives, doxorubicin, doxorubicin derivatives, bleomycin, plicomycin, duocarmycin SA, duocarmycin CN, duocarmycin DMG, duocarmycin DMA, duocarmycin MA, duocarmycin TM, duocarmycin MB, duocarmycin GA, tomaymycin, illudin, irofulven, apaziquone, triptolide, auristatin, auristatin E and monomethyl auristatin E, methotrexate, platinum drugs and derivatives thereof ,pharmaceutically acceptable salts thereof and mixtures thereof or the prodrugs are selected from the group comprising INNO-206 (a 6-maleimidocaproyl hydrazone derivative of doxorubicin), cyclophosphamide, capecitabine, dacarbazine, prodrugs of 5-fluorouracil, etoposide phosphate prodrugs, gemcitabine phosphoramidate prodrug, irinotecan and mixtures thereof or the antigens are selected from the group comprising WT1,MUC1, LMP2, HPV E6, E7, EGFRvIII, HER-2/neu, Idiotype, MAGE A3, p53 nonmutant, NY-ESO-1, PSMA, GD2, CEA, MelanA/MART1, Ras mutant, gp100, p53 mutant, Proteinase3, bcr-abl, Tyrosinase, Survivin, PSA, hTERT, Sarcoma translocation breakpoints, EphA2, PAP, ML-IAP, AFP, EpCAM, ERG, NA17, PAX3, ALK, androgen receptor, Cyclin B1, Polysialic acid, MYCN, RhoC, TRP-2, GD3, Fucosyl GM1, Mesothelin, PSCA, MAGE A1, sLe, CYP1b1, PLAC1, GM3m, BORIS, Tn, MAGE-B2, DAM-10, SAGE, RAGE and mixtures thereof.

5. The multivalent conjugate according to any one of Claims 1 to 4, wherein the gold nanostructure (GNS) are functionalized with sulfhydryl groups, methyl groups, carboxylic acid groups or amine groups.

6. The multivalent conjugate according to any one of Claims 1 to 5, wherein said functionalized gold nanostructures have present at their surface from 1 to 100,000 amine groups.

7. The multivalent conjugate according to any one of Claims 1 to 6, wherein said functional equivalent of the Shiga B toxin is selected from the group of Shiga-like toxin 1, Shiga-like toxin 2 , Shiga-like toxin 2c, Shiga-like toxin 2d1, Shiga-like toxin 2d2, Shiga-like toxin 2e, Shiga-like toxin 2f and Shiga-like toxin 2y,verotoxin-1, verotoxin-2, verotoxin 2c or verotoxin 2v.

8. The multivalent conjugate according to any one of Claims 1 to 7, for use in distinguishing normal cells from abnormal cells.

9. The multivalent conjugate according to Claim 8, wherein said normal cells and abnormal cells are distinguished by magnetic resonance imaging or near-infrared fluorescence imaging.

10. The multivalent conjugate according to any one of Claims 1 to 7, for use as a medicament.

11. The multivalent conjugate according to Claim 10 for use in treating cancer cells.

12. The multivalent conjugate according to any one of Claims 8 to 10, wherein said cancer cells are distinguished using one of the following:
(1) photothermal therapy,
(2) imaging using near-infrared fluorescence or MRI or Positron emission tomography (PET) or single photo emission computed tomography (SPECT),
(3) photothermal therapy and administering cytotoxic agents or vice versa,
(4) imaging using near-infrared fluorescence or MRI and administering cytotoxic agents or vice versa,
(5) photothermal therapy and administering cancer antigens or vice versa, or
(6) imaging using near-infrared fluorescence or MRI and administering cancer antigens or vice versa.

13. The multivalent conjugate according to any one of Claims 1 to 7, for use in imaging cancer cells.

14. The multivalent conjugate according to any one of Claims 10 to 13, wherein said cancer cells are selected from the group comprising acute lymphoblastic leukemia, acute myeloid leukemia, adrenocortical carcinoma, Kaposi sarcoma, lymphoma, anal cancer, appendix cancer, astrocytomas, atypical teratoid/rhabdoid tumor, basal cell carcinoma, bile duct cancer, bladder cancer, bone cancer, brain stem glioma, brain tumor, breast cancer, bronchial tumors, Burkitt lymphoma, carcinoid tumor, cardiac tumors, cervical cancer, chordoma, chronic lymphocytic leukemia, chronic myelogenous leukemia, chronic myeloproliferative disorders, colon cancer, craniopharyngioma, cutaneous T-cell lymphoma, extrahepatic cancer, ductal carcinoma *in situ,* embryonal tumors, endometrial cancer, ependymoma, esophageal cancer, esthesioneuroblastoma, Ewing sarcoma, extracranial germ cell tumors, extrahepatic bile duct cancer, eye cancer, fibrous histiocytoma of the bone, gallbladder cancer, gastric cancer, gastrointestinal carcinoid tumors, gastrointestinal stromal tumors, germ cell tumors, gestational trophoblastic tumors, glioma, hairy cell leukemia, head and neck cancer, heart cancer, hepatocellular cancer, histiocytosis, Hodgkin lymphoma, hypopharyngeal cancer, intraocular melanoma, islet cell tumors, kidney cancer, Langerhans cell histiocytosis, laryngeal cancer, leukemia, lip and oral cavity cancer, liver cancer, lobular carcinoma *in situ,* lung cancer, lymphoma, macroglobulinemia, male breast cancer, malignant fibrous histiocytoma of bone and osteosarcoma, melanoma, Merkel cell carcinoma, mesothelioma, metastic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, ovarian cancer, pancreatic cancer, papillomatosis, paraganglioma, paranasal sinus and nasal cavity cancer, parathyroid cancer penile cancer, pharyngeal cancer, pheochromocytoma, pituitary tumors, plasma cell neoplasma, pleuropulmonary blastoma, primary central nervous system lymphoma, prostate cancer, rectal cancer, renal cell cancer, renal, pelvis and ureter transitional cell cancer, retinoblastoma, rhabdomyosarcoma, salivary gland cancer, sarcoma, Sézary syndrome, skin cancer, small lung cancer, small intestine cancer, soft tissue sarcoma, squamous cell carcinoma, squamous neck cancer with occult primary, metastatic, stomach cancer, T-cell lymphoma, testicular cancer, throat cancer, thymoma and thymic carcinoma, transitional cell cancer of the renal pelvis and ureter, trophoblastic tumor, ureter and renal pelvis cancer, urethral cancer, uterine cancer, uterine sarcoma, vaginal cancer and vulvar cancer.

15. A pharmaceutical composition comprising a first multivalent conjugate according to any one of Claim 1 to 7, wherein T is a cytotoxic agent or a contrast agent and a second multivalent conjugate according to any one of Claims 1 to 9, wherein T is a cancer antigen and a pharmaceutically acceptable vehicle.
